# EUROPEAN PATENT SPECIFICATION

(11) **EP 1 373 570 B1**
(45) Date of publication and mention of the grant of the patent: **12.09.2007**
(21) Application number: 02715053.1
(22) Date of filing: 07.03.2002
(51) Int. Cl.: C12P 19/34, C07H 21/02, C07H 21/04, C12P 21/00

(54) **IMPROVED IN VITRO SYNTHESIS SYSTEM**
VERBESSERTES IN-VITRO-SYNTHESESYSTEM
SYSTEME DE SYNTHESE IN VITRO AMELIORE

(30) Priority: 08.03.2001 US 273827 P
(43) Date of publication of application: 02.01.2004
(73) Proprietor: INVITROGEN CORPORATION, Carlsbad, CA 92008 (US)
(72) Inventor: CHATTERJEE, Deb, K., North Potomac, MD 20878 (US); LONGO, Mary, C., Germantown, MD 20874 (US)
(74) Representative: Couchman, Jonathan Hugh
(86) International application number: PCT/US2002/006812
(87) International publication number: WO 2002/072890

(56) References cited:
- WO-A1-00/55353
- JP-A- 7 000 194
- US-A- 5 665 563
- RYABOVA LYUBOV A ET AL: "Acetyl phosphatase as an energy source for bacterial cell-free translation systems" ANALYTICAL BIOCHEMISTRY, vol. 226, no. 1, 1995, pages 184-186, XP002319200 ISSN: 0003-2697
- MURO-PASTOR A M ET AL: "The nuiA Gene from Anabaena sp. encoding an inhibitor of the NucA sugar-non-specific nuclease" JOURNAL OF MOLECULAR BIOLOGY, LONDON, GB, vol. 268, no. 3, 9 May 1997 (1997-05-09), pages 589-598, XP004459777 ISSN: 0022-2836
- WILLIAMS J G K ET AL: "PARTIAL PURIFICATION AND PROPERTIES OF AN EXONUCLEASE INHIBITOR INDUCED BY BACTERIOPHAGE MU-1" JOURNAL OF VIROLOGY, THE AMERICAN SOCIETY FOR MICROBIOLOGY, US, vol. 39, no. 2, August 1981 (1981-08), pages 548-558, XP001087798 ISSN: 0022-538X

### SEQUENCE LISTING

<110> Invitrogen Corporation
<120> Improved In Vitro Synthesis System
<130> 0942.525PC01
<160> 5
<170> Patent In version 3.1
<210> 1
   <211> 12
   <212> DNA
   <213> Artificial Sequence
<220>
   <223> Shine-Dalgarno sequence
<400> 1
   aggagttcag cc 12
<210> 2
   <211> 32
   <212> DNA
   <213> Artificial Sequence
<220>
   <223> DE230 primer
<400> 2
   gggaggccat ggatattaat actgaaactg ag 32
<210> 3
   <211> 37
   <212> DNA
   <213> Artificial Sequence
<220>
   <223> DE231 primer
<400> 3
   gggaggagat ctttatacct ctgaatcaat atcaacc 37
<210> 4
   <211> 14
   <212> DNA
   <213> Artificial Sequence
<220>
   <223> Engineered optimal Shine-Dalgarno sequence
<400> 4
   aggaggaatt aacc 14
<210> 5
   <211> 42
   <212> DNA
   <213> Artificial Sequence
<220>
   <223> DE255 primer
<400> 5
   gggaggccat gggaaacgct tattacattc aggatcgtct tg 42

## Description

### BACKGROUND OF THE INVENTION

### Field of the Invention

The present invention relates to *in vitro* production of protein from input mRNA or from input DNA that is transcribed and/or translated efficiently to produce protein in improved quantities. The present invention also relates to *in vitro* production of nucleic acid molecules.

### Related Art

*In vitro* protein synthesis has among its advantages specifically producing the desired protein without unnecessarily producing undesired proteins that are required for maintaining cells used for protein production in *in vivo* or cellular systems for protein synthesis. When a cell is used as a protein factory, in addition to producing the desired protein, the cell produces the other necessary molecules, including undesired proteins, that are required to maintain the cell. Cell-free systems are very popular because there are standard protocols available for their preparation and because they are commercially available from a number of sources.

Because it is essentially free from cellular regulation of gene expression, *in vitro* protein synthesis has advantages in the production of cytotoxic, unstable, or insoluble proteins. The over-production of protein beyond a predetermined concentration can be difficult to obtain *in vivo,* because the expression levels are regulated by the concentration of product. The concentration of protein accumulated in the cell generally affects the viability of the cell, so that over-production of the desired protein is difficult to obtain. In an isolation and purification process, many kinds of protein are insoluble or unstable, and are either degraded by intracellular proteases or aggregate in inclusion bodies, so that the loss rate is high. *In vitro* synthesis circumvents many of these problems. Also, through simultaneous and rapid expression of various proteins in a multiplexed configuration, this technology can provide a valuable tool for development of combinatorial arrays for research, and for screening of proteins. In addition, various kinds of unnatural amino acids can be efficiently incorporated into proteins for specific purposes (Noren et al, Science 244: 182-188 (1989)). However, despite all its promising aspects, the *in vitro* system has not been widely accepted as a practical alternative, in part due to the short reaction period, which causes a poor yield of protein synthesis.

Protein synthesis is guided by an RNA polynucleotide template that encodes the desired protein. Protein synthesis can be initially guided by a DNA template that is transcribed to produce the RNA polynucleotide that encodes the desired protein. The DNA template therefore minimally includes the DNA to be transcribed as well as a binding site for RNA polymerase that catalyzes transcription of the mRNA template that is translated to produce protein. When the transcription and translation are coupled in one system, the system is called an *In Vitro* Transcription Translation system (IVTT).

IVTT or protein synthesis using cell-free extracts is becoming an important tool for analysis of proteins. The availability of complete genome sequences provides a wealth of information on the molecular structure and organization of a myriad of genes and open reading frames whose functions are not known or are only poorly understood. Thus, the utility of IVTT and more generally, protein synthesis *in vitro,* is expected to be even more important in the future for rapid and efficient protein synthesis and functional analysis.

The techniques of modern molecular biology have made possible manipulation of DNA as well as the other cellular components expressed from the genes contained in the DNA of an organism. In living cells, DNA is transcribed to make mRNA which is then used as a template for translation whereby a protein whose sequence is determined by the DNA is made. In developing the modern tools of molecular biology, research has been directed toward ways to perform various steps of the transcription and/or translation processes *in vitro* under controlled conditions and with defined inputs. These procedures mimic, in essence, similar processes that occur in a much more heterogeneous mixture in living cells.

Even before the advent of modern recombinant technology, cell extracts were developed which allowed the synthesis of protein *in vitro* from purified mRNA transcripts. To date, several systems have become available for the study of protein synthesis and RNA structure and function. To synthesize a protein under investigation, a translation extract must be "programmed" with an mRNA corresponding to the gene and protein under investigation. The mRNA can be produced from DNA, or the mRNA can be added exogenously in purified form. Historically, such mRNA templates were purified from natural sources or, using more recently developed technologies, prepared synthetically from cloned DNA using bacteriophage RNA polymerases in an *in vitro* reaction.

More recently, techniques using coupled or complementary transcription and translation systems which carry out the synthesis of both RNA and protein in the same reaction have been developed (IVTT). The cell extracts used for the modern techniques must contain all the components necessary both for transcription (to produce mRNA) and for translation (to synthesize protein) in a single system. In such a system, the input template is DNA, which is normally much easier to obtain than RNA and much more readily manipulable.

An early coupled system was based on a bacterial extract (Lederman and Zubay, Biochim. Biophys. Acta, 149: 253 (1967). Since prokaryotes normally carry out a coupled reaction within their cytoplasm, this bacterial based system closely reflected the *in vivo* process. This general system has seen widespread use for the study of prokaryotic genes. However, this general bacterial system is generally not useful for eukaryotic genes, due to its inefficiency and relatively high nuclease content.

Nucleases are phosphoidesterases with various substrate requirements. Nucleases are classified by their specificity as exo- or endo- nucleases that cleave phosphodiester bonds, such as those bonds forming polynucleotides of DNA or RNA. DNases cleave phosphodiester bonds in DNA molecules. RNases cleave phosphodiester bonds in RNA molecules.

Exonucleases require a free end to start cleaving the polynucleotide strand, while endonucleases cleave from within a polynucleotide molecule even when no free ends are available, for example, in a covalently closed polynucleotide circle. Some nucleases have both, endo-and exo-activities. Some are specific for single-stranded DNA (ss-DNA) others for double-stranded DNA (ds-DNA). Several nucleases can cleave both types of DNA.

Some exonucleases work in a 3' to 5' direction, while others function in a 5' to 3' direction. Some are not direction specific and work in both directions. Some endonucleases are nucleotide sequence or site-specific in their recognition sites for cleavage. Also, there are nucleases that exhibit other non-cleavage functions on the same protein or complex as the protein with nuclease activity. Sometimes different functions can be attributed to specific functional domains. In this application, "nuclease" refers to nucleases in general unless the specific context indicates a specific nuclease or a nuclease with a specific activity, such as, but not limited to, endo- activity or DNase activity.

In addition to prokaryotic system extracts, eukaryotic system extracts have also been developed. These eukaryotic systems use exogenously added *E.coli* RNA polymerase or wheat germ RNA polymerase to transcribe exogenous DNA. These systems have had limited success for the general study of eukaryotic genes, due to their low efficiency, and to the fact that they were developed and used prior to the widespread success of cDNA cloning techniques. Other coupled systems have been developed for the study of viral protein synthesis, but are not generally useful for non-viral templates.

In the mid-1980s, the development of more efficient *in vitro* transcription systems, particularly ones using phage polymerases such as T7, SP6 and T3, allowed protein synthesis systems to be defined that more efficiently translated cloned mRNA sequences *in vitro* using translation extracts from wheat germ and rabbit reticulocytes. For example, Perara and Lingappa (J. Cell Biol., 101: 2292-2301 (1985)) showed that SP6 RNA polymerase transcription reactions could be added directly to reticulocyte lysate for the production of protein.

This insight illuminated the need to purify the mRNA prior to translation. Later other workers showed that the transcription and translation could be coupled in reticulocyte lysate by including a phage polymerase and appropriate transcriptional co-factors in the reaction (Spirin et al, Science, 242: 1162-1164 (1988); Craig et al, Nucleic Acids Res., 20: 4987-4995 (1992)). More recently, U.S. Pat. No. 5,324,637 to Thompson et al described a coupled transcription and translation system in eukaryotes. Thompson used reticulocyte lysate and a phage polymerase where the coupling of the two reactions was facilitated by specific conditions, notably the concentration of magnesium ions, which permitted both transcription and translation to occur in the same reaction mix.

Although the coupled approach for transcription and translation systems is useful for many proteins, translation efficiencies vary widely depending on the type of DNA template which is used (e.g., supercoiled plasmid DNA or linear DNA). In addition, the amount of mRNA synthesized in a coupled reaction is difficult to control under most coupled conditions, such as disclosed in Thompson. Since efficiency and fidelity of translation are dependent upon the amount of mRNA added to and present during the reaction, a possible explanation for the undesirable variability of results obtained using these coupled systems, in which the reactions occur simultaneously, is that transcription and translation are not consistent between various templates under the conventional reaction conditions.

A number of subsequent improvements have been made (see e.g., Kim et al, Eur. J. Biochem., 239: 881-886, (1996); Patnaik and Swartz, Biotechniques, 24: 862-868, (1998); and Kim and Swartz, Biotech. and Bioeng., 66: 180-188, (1999)) to improve the IVTT system. One of the main problems, however, of the conventional IVTT systems is that these systems do not produce sufficient quantities of protein for extensive analysis of protein(s) of interest.

The conventional inefficient protein synthesis can be in part attributed to factors such as maintenance of an energy supply, the stability of the DNA template for transcription and the stability of mRNA for translation.

The problem of DNA template stability is especially evident when linear substrates, such as PCR derived products or restriction enzyme(s) digested fragments, are used in cell-free extracts for generating protein(s). The linear DNA fragments are susceptible to rapid degradation by intracellular exonucleases of *E.coli,* particularly RecBCD (Pratt et al, Nucleic Acids Res., 9: 4459-4474, (1981); Benzinger et al, J. Virol., 15: 861-871, (1975); Lorenz and Wackernagel, Microbiol Rev., 58, 563-602, (1994)) and possibly by other nucleases.

In most cases, a supercoiled plasmid DNA containing the gene of interest is used in IVTT systems because plasmid DNAs are more stable (Kudlicki et al, Anal. Biochem., 206: 389-393, (1992)). Linear DNAs are more readably degraded by DNA nucleases, especially DNA exonucleases, such as RecBCD. Mutant RecBCD strains devoid of the exonuclease have been made. These mutant strains do not so rapidly degrade linear DNA; however, such mutant strains grow extremely poorly and therefore do not produce satisfactory results (Yu et al, PNAS, 97: 5978-5983, (2000)).

*E.coli* extract for cell-free protein synthesis has been made using a RecD mutant of *E.coli* (Lesley et al, J. Biol. Chem., 266: 2632-2639, (1991)). However, cell-free extract made using RecD mutant *E.coli* contained high level of chromosomal DNA contamination because sheared chromosomal DNA is not degraded by the nuclease that has been mutated. To remedy this, micrococcal nuclease has been added to degrade the contaminating chromosomal DNA to minimize background. Similarly, entire RNase E deletion mutants have been made, but cell growth of these complete deletion mutants is also poor and unsuitable for providing a cell free extract.

In addition, the cell-free extract made from such mutants did not enhance protein production and for unknown reason, the protein synthesis is independent of T7 RNA polymerase addition even though the PCR product contained T7 promoter.

*E.coli* also contains deoxyribonucleases (DNases) (Linn and Deutscher, n: Nucleases, Cold Spring Harbor Laboratory Press, 455-468, (1993)) with specificity for double stranded DNA. These nucleases may be also responsible for degradation of the template DNA and thereby reduce protein production.

As discussed above, the insufficient protein synthesis in a coupled IVTT system can result either from the instability of DNA or from the instability of mRNA. In non-coupled synthesis systems insufficient protein synthesis can result especially from instability of mRNA.

*E.coli* is known to contain a large number of RNases (Linn and Deutscher, In: Nucleases, Cold Spring Harbor Laboratory Press, 455-468, (1993)). RNase I, a periplasmic enzyme is one the major non-specific RNases in *E.coli* that acts on oligo RNA as a substrate (Meador et al, *Eur. J. Biochem.,* 187: 549, (1990); and Meador and Kennel, *Gene,* 95: 1, (1990)). Several RNases participate in mRNA degradation in *E.coli,* including endonucleases (such as RNase E, RNase K and RNase III) and 3'-exonucleases such as RNase II and polynucleotide phosphorylase (Gros et al, Nature, 190: 581-585, (1961); Emory et al, Genes Dev., 6: 135-148, (1992); Belasco, J. G., Control of mRNA stability, Academic Press, 3-12, (1993); Lopez et al, Mol. Microbiol., 33: 188-199, 1999; Mohanty and Kushner, PNAS, 97: 11966-11971, (2000)). The present invention is in part based on a premise that mutating or inhibiting these enzymes might therefore enhance protein synthesis in cell-free extracts.

RNase mutants are known in the art. See for example, Niyogi and Datta, J. Biol. Chem., 250: 7307-12 (1975) wherein *E.coli* deficient in RNase I, RNase II and RNase III were used for characterization of a novel ribonuclease. An RNase I deficient mutant has been used as a potential source of material for IVTT, but with unsatisfactory results with respect to efficient protein production (Kudlicki et al, Anal. Biochem., 206: 389-393 (1992); and Ellman et al, Methods in Enzymology, 202: 301-336 (1991)). Methods for mutating and selecting other mutants, for example, nuclease mutants, are known in the art.

The inefficient protein synthesis resulting from conventional synthesis systems can also be in part attributed to factors relating to the maintenance of amino acid and energy (fuel) supplies to support the synthesis reaction.

WO 00/55353 discloses two methods for replenishing ATP necessary for translation. According to these methods, PEP (phosphoenolpyruvate) or pyruvate is used to regenerate the ATP energy source. The first disclosed method was previously known in the art and involves phosphoenolpyruvate (PEP) used in conjunction with pyruvate kinase to regenerate ATP from ADP. In the second method of WO 00/55353, pyruvate is used in conjunction with pyruvate oxidase to regenerate ATP.

Both energy sources and amino acids are depleted in these systems irrespective of protein synthesis (WO/0055353; Kim and Choi, J. Biotech., 84: 27-32, (2000)). Protein synthesis could be restored by a second addition of amino acids and the energy source.

Although these methods may be useful for regenerating ATP, inefficient use of the ATP initially available or generated can still result in reduced protein yields. For example, phosphatases can hydrolyze the ATP, thus making ATP energy unavailable for the desired IVTT process. The alkaline phosphatase of *E.coli* is one of the known phosphatases that can hydrolyze ATP (see, for example, Enzymology Primer for Recombinant DNA Technology, by Hyone-Myong Eun, Academic Press, pages 307-333). Many enzymes with names not including "phosphatase", e.g., "helicase", also hydrolyze ATP. Therefore, the ATP that is being made from the energy generating system with a goal of driving protein synthesis can potentially be wasted by active phosphatases, such as an alkaline phosphatase or a helicase, with a resultant reduction of protein synthesis.

It has been shown in a wheat germ cell-free system that phosphatase-immunodepletion improved protein synthesis by reducing ATP and GTP hydrolysis (Kawarasaki et al, J. Biotech. 61: 199-208, (1998)). It has also been suggested that phosphoenolpyruvate (PEP), the substrate generally used for regeneration of ATP, is also degraded by phosphatase(s) limiting protein synthesis (Kim and Swartz, Biotech. and Bioeng., 66: 180-188, (1999); Swartz and Kim, USP 6,168,931 (2001); Kim and Choi, J. Biotech. 84: 27-32, (2000)).

In view of the present state of the art, there is a need to develop an improved IVTT system that will enhance the production of protein(s). The present invention meets this need by virtue of providing compositions and methods for stabilizing or maintaining template DNA, by stabilizing or maintaining mRNA including mRNA derived from the template(s), by conserving energy to be used for synthesis and/or by providing sufficient energy regenerating substrates to provide the energy necessary for efficient protein synthesis from the templates.

All publications and patent applications referenced in this specification are hereby each incorporated in their entirety by reference.

### SUMMARY OF THE INVENTION

The present invention relates to *in vitro* synthesis, more specifically to *in vitro* peptide/protein or nucleic acid synthesis systems, and to methods and kits that improve efficiency of such *in vitro* synthesis and related compositions. More specifically, the present invention features systems, methods and kits that improve efficiency of protein or nucleic acid synthesis by providing an improved energy supply for synthesis and/or by maintaining the nucleic acid template(s) for synthesizing proteins or nucleic acids for a more efficient and extended synthesis reaction. The present invention may be used in any type of *in vitro* protein synthesis system, including coupled transcription/translation systems and uncoupled translation systems. Preferred synthesis systems of the invention comprise at least one cell extract, at least one energy source, and at least one nucleic acid template.

Compositions and methods are provided to enhance the synthesis with cell extracts. In one aspect, the cell providing the extract or components for synthesis is modified or mutated to inhibit or inactivate unwanted components/proteins/enzymes in the synthesis reaction. For example, mutations can be made in RNases, such as RNase E, or in other enzymes, such as alkaline phosphatase and endonuclease A in accordance with the present invention. In addition, inhibitors, such as inhibitors of nucleases that act on nucleic acid templates (e.g., Gam protein of phage lambda to inhibit RecBCD) or inhibitors of other unwanted or detrimental components/proteins/enzymes in the synthesis reaction can be used to enhance the production of desired products *in vitro.*

Alternatively, or in addition to the modified/mutant cell extracts and/or inhibitors, synthetic pathways of deleterious components/proteins/enzymes can be shut down or slowed, for example, by growth strategies that fail to induce the normal quantities of deleterious components/proteins/enzymes or that fail to provide cofactors for the enzymatic activity, or by inhibitors of transcription or translation of the deleterious genes or proteins. Employing growth strategies that result in diminished enzyme activity is one embodiment of the present invention for modulating or inhibiting enzymes. The present invention also relates to these modified/mutated cells and/or genes and media capable of growing these cells.

Thus, the present invention more generally relates to more efficient synthesis involving, for example, at least one extract from a cell from which at least one enzyme/component/protein that participates in hydrolysis of high energy phosphate bonds or hydrolysis or formation of phosphodiester bonds is absent, is removed, or is modified to reduce or inactivate its activity (for example, by modulating, mutating, or modifying one or more genes involved in expression or which encode such protein/component/enzyme); at least one inhibitor of at least one enzyme/component/protein that participates in hydrolysis of high energy phosphate bonds or hydrolysis or formation of phosphodiester bonds; and/or at least two sources of energy to provide chemical energy for the synthesis process. In accordance with the invention, any one or a number of these features may be combined and used in various in *vitro* synthesis systems. As will be recognized, the invention may also be used to prepare various intermediates of a protein synthesis reaction, if desired. For example, the invention may be used to prepare RNA (during transcription of a DNA template) and such RNA may be isolated or further processed by standard molecular biology techniques.

More specifically, the present invention features embodiments that include an *in vitro* synthesis system that includes one or more (or combinations thereof) of the following: i) removal, modulation, inhibition or inactivation of at least one activity, e.g., an enzymatic activity, that catalyzes hydrolysis of high energy phosphate bonds or hydrolysis or formation of phosphodiester bonds, for example, an enzyme selected from the group consisting of a nuclease, a phosphatase and a polymerase; ii) use of at least one cell extract for synthesis, wherein the extract is modified to exhibit reduced activity (compared to an unmodified extract) or inactivation of at least one enzyme that catalyzes hydrolysis of high energy phosphate bonds or hydrolysis or formation of phosphodiester bonds, for example, an enzyme selected from the group consisting of a nuclease, a phosphatase and a polymerase; and iii) at least two energy sources that supply energy for synthesis.

The present invention also features compositions for carrying out the invention and to compositions made while carrying out the invention. Such compositions may comprise any one or a combination of the elements of the invention (e.g. inhibitors, cell extracts, energy sources, etc.) and/or they may also comprise substrates used during transcription and/or translation reactions (e.g. nucleotides, amino acids, polymerases, enzymes, cofactors, buffers and buffering salts). In addition, such compositions may comprise any number of products of the transcription and/or translation reaction such as RNA, peptides, proteins, etc. Preferably, the compositions of the invention may comprise at least one component selected from the group consisting of at least one inhibitor of at least one enzyme, e.g., an enzyme selected from the group consisting of a nuclease, a phosphatase and a polymerase; at least one extract for protein synthesis, wherein the extract is modified to exhibit reduced activity of at least one function selected from the group consisting of, e.g., a nuclease, a phosphatase and a polymerase; and at least two energy sources that supply energy for synthesis.

The present invention also features improved methods for in *vitro* synthesis. Such methods provide for the production of various products in *vitro* including RNA or other nucleic acid molecules, and peptides or proteins. In one aspect, RNA or other nucleic acid molecules may be produced by mixing one or more nucleic acid templates and at least one component of the invention (e.g. inhibitors, energy sources, cell extracts, etc.), and incubating said mixture under conditions sufficient to produce one or more nucleic acid molecules (e.g. RNA) complementary to all or a portion of said template. In another aspect, peptides or proteins may be produced by mixing one or more nucleic acid templates (preferably RNA) and at least one component of the invention (inhibitors, energy sources, etc.) and incubating said mixture under conditions sufficient to produce one or more peptides or proteins encoded by all or a portion of said template. The methods of the invention may further comprise additional steps for further processing the products produced. For example, the produced nucleic acid molecules and proteins may be used to produce other products. They may be used in activity or functional assays or they may be further purified. As will be appreciated, the methods of the invention may also be carried out in the presence of one or more other components such as one or more nucleotides or derivatives thereof (which may be detectably labeled), one or more amino acids or derivatives thereof, one or more polymerases, one or more cofactors, one or more buffers and/or buffering salts, one or more energy sources (ATP, PEP, etc.), one or more cell extracts, one or more nucleic acid templates and the like. The methods of the invention preferably include one or more components of the invention (or combinations thereof) selected from the group consisting of at least one inhibitor of at least one enzyme, e.g., an enzyme selected from the group consisting of a nuclease, a phosphatase and a polymerase; at least one extract for synthesis, wherein the extract is modified to exhibit reduced activity of at least one enzyme e.g., an enzyme selected from the group consisting of a nuclease, a phosphatase and a polymerase; and at least two energy sources that supply energy for synthesis. The method preferably entails contacting one or more nucleic acid templates with at least one component selected from the group consisting of: at least one extract from a cell from which at least one enzyme/component/protein is absent, is removed, or is modified or mutated to reduce or inactivate its activity that catalyzes hydrolysis of high energy phosphate bonds or hydrolysis or formation of phosphodiester bonds or inactivate its activity; at least one inhibitor of at least one enzyme/component/protein that catalyzes hydrolysis of high energy phosphate bonds or hydrolysis or formation of phosphodiester bonds; and at least two energy sources providing chemical energy for synthesis, to form a mixture; and incubating the mixture under conditions sufficient to produce at least one protein encoded by all or a portion of templates. If desired, any detection method known in the art can be used to monitor product (e.g. RNA and protein) production. Inhibition of an enzyme can be accomplished by selected growth conditions in place of or in addition to adding a chemical inhibitor. The inhibitor can also be synthesized *in situ.* For example, one or more genes encoding at least one inhibitor (e.g., Gam) can be expressed in a cell or cell culture used to prepare a cell extract used in the invention. Accordingly, any number of desired inhibitors may be provided to the *in vitro* reaction mixture without the need for separate addition. In one aspect, such inhibitors are produced recombinantly, e.g., at least one inhibitor gene is cloned and expressed.

More specifically, the present invention relates to methods of an in *vitro* synthesis that includes at least one or at least two or at least three components selected from the group consisting of at least one inhibitor of at least one enzyme, e.g., an enzyme selected from the group consisting of a nuclease, a phosphatase and a polymerase; at least one cell or extract for synthesis, wherein the cell or extract is modified from a native or wild type extract to exhibit reduced activity of at least one enzyme, e.g., an enzyme selected from the group consisting of a nuclease, a phosphatase and a polymerase; and at least two energy sources that supply energy for protein and/or nucleic acid synthesis.

Kits for *in vitro* synthesis are also a feature of the present invention. Such kits may contain any number or combination of reagents or components for carrying out the invention. Kits of the invention preferably comprise one or more elements selected from the group consisting of one or more components of the invention (e.g. inhibitors, energy sources, cell extracts, etc.) one or more nucleotides or derivatives thereof, one or more amino acids or derivatives thereof, one or more polymerases, one or more cofactors, one or more buffers or buffer salts, one or more energy sources, one or more cell extracts, one or more nucleic acid templates, one or more reagents to determine the efficiency of the kit or assay for production of the products such as nucleic acid and protein products, and directions or protocols for carrying out the methods of the invention or to use of the kits of the invention and/or its components. The kit of the invention may comprise one or more of the above components in any number of separate containers, tubes, vials and the like or such components may be combined in various combinations in such containers. More specifically, the kits of the invention may comprise at least one component selected from the group consisting of at least one inhibitor of at least one enzyme, e.g., an enzyme selected from the group consisting of a nuclease, a phosphatase and a polymerase; at least one extract for protein synthesis, wherein the extract is modified to exhibit reduced activity of at least one enzyme, e.g., an enzyme selected from the group consisting of a nuclease, a phosphatase and a polymerase; and at least two energy sources that supply energy for synthesis.

Many nucleases are known which can be removed, modulated, mutated or inhibited in accordance with the present invention. In one aspect, genes encoding such nucleases can be mutated or modified in the cells from which an *in vitro* synthesis extract is made. Many such genes are known or can be readily identified and modified by techniques well known in the art or described herein. Furthermore, expression of such nucleases can be modulated by controlling growth conditions of a cell. Moreover, compounds/molecules/proteins can be used to inhibit one or more of such nucleases to prevent such nucleases from impairing synthesis. Such inhibitors are known or can be readily identified for use in the invention. By starting with these mutated or selectively grown cells or by adding or including inhibitors of nucleases to the synthesis system, superior synthesis results can be obtained.

Inhibitors can be used or included in the systems of the invention by any known method. For example, inhibitors may be added to the system before, during or after introduction of the nucleic acid template. Inhibitors can also be transcribed or expressed in a cell used to prepare the extract or transcribed or expressed during the protein synthesis reaction. Although inhibitors may be biosynthetic compounds, inhibitors of the invention are not limited to compounds that can be produced biologically.

Examples of nucleases that can be removed, inhibited, mutated, modified, or modulated according to the invention include: exonuclease I, exonuclease II, exonuclease III, DNA polymerase II, DNA polymerase III (ε subunit), exonucleases IVA and IVB, RecBCD (exonuclease V), exonuclease VII, exonuclease VIII, RecJ, dRpase, endonuclease I, endonuclease III, endonuclease IV, endonuclease V, endonuclease VII, endonuclease VIII, fpg, uvrABC, mutH, vsr endonuclease, ruvC, ecoK, ecoB, mcrBC, mcrA, mrr, and topoisomerases (such as topoisomerase I, topoisomerase II, topoisomerase III and topoisomerase IV). Such removal, inhibition, etc., allows preservation or protection of the nucleic acid template used in the synthesis reactions of the invention. For example, DNA nucleases of cells can be mutated, modified, inhibited, etc. to maintain or preserve the DNA templates. Such DNases from *E.coli* and other cells are known in the art.

Additionally, RNA nucleases can be mutated, modified, inhibited, etc. to protect or preserve the RNA template. For example, *E.coli* ribonucleases, such as endoribonuclease I, M, R, III, P, E, K, H, HII, IV, F, N, P2, 0, PC and PIV, and exonucleases such as polynucleotide phosphorylase, oligoribonuclease, and exoribonucleases II, D, BN, T, PH and R, can be mutated or modified or inhibited to protect mRNA for protein synthesis. Depending on the cell used for the extract, other ribonucleases native to that cell can be mutated, removed, modified, or inhibited, etc. to maintain or protect the template(s) for protein synthesis. Many nucleases and nuclease inhibitors are commercially available. For example, Rnasin® (Promega), is well characterized as an RNase inhibitor in mammalian systems, but is not effective in inhibiting prokaryotic RNases.

Methods for mutating genes and selecting mutated genes encoding nucleotides are known in the art. Such mutations include point mutations (for example, by site directed mutagenesis), deletion mutations and insertion mutations. The invention also contemplates the use of the aspects of the invention including the mutation(s), modification(s), inhibitor(s) and other methods singly, or preferably, in combination. These embodiments relating to removal, modulation or reduction of enzymatic activity can further include features that support the energy requirements for the synthesis reaction.

Removal, modulation or reduction of other enzymes can also be useful to the extent that they direct or accentuate production of the desired product. For example, when the nucleic acid template is an RNA, polymerase activity may be undesirable. Inactivation or inhibition of polymerase activity may therefore improve yields and purity of the desired protein product.

Modulation of RNA polymerase may also be helpful in systems using a DNA template to produce RNA. When RNA synthesis is rapid, the RNA may be insufficiently protected by ribosomes. Use of a mutated or modulated RNA polymerase can advantageously spare the RNA by allowing ribosomes proper time to bind and protect the nascent RNA.

Protein and nucleic acid synthesis typically requires an energy source. It is thus a feature of the present invention to provide a sufficient energy source to support such synthesis. Energy is required for initiation of transcription to produce mRNA (e.g., when a DNA template is used and for initiation of translation high energy phosphate for example in the form of GTP is used). Each subsequent step of one codon by the ribosome (three nucleotides; one amino acid) requires hydrolysis of an additional GTP to GDP. ATP is also typically required. For an amino acid to be polymerized during protein synthesis, it must first be activated. Activation requires hydrolysis of two high energy phosphate bonds. Thus an amino acid monomer in the presence of Mg⁺² tRNA and ATP reacts to form an aminoacyl-tRNA, AMP and PP*ᵢ*. Significant quantities of energy from high energy phosphate bonds are thus required for protein and/or nucleic acid synthesis to proceed.

The present inventors have found that improved synthesis is achievable using more than one, preferably two or more energy sources that can provide the chemical metabolites necessary for synthesis. Energy sources that generate or regenerate high energy phosphate bonds are preferred.

Preferably, the present invention features one or more of the above components or methods to improve efficiency of nucleic acid and/or protein synthesis. Combinations of these improvements are also embodiments of the present invention. For example, a composition, a system, a method or a kit of the invention may feature any one or a combination of improvements described herein, for example: at least one energy source and removal and/or modulation of at least one enzyme activity; at least one energy source and at least one inhibitor; removal and/or modulation of at least one enzyme activity and one inhibitor; at least one energy source, removal and/or modulation of at least one enzyme activity and one inhibitor; at least two energy sources; removal and/or modulation of at least two enzyme activities; and at least two inhibitors. More generally, in accordance with the invention at least one type of a first improvement is combined with at least two types of a different improvement; three or more types of any improvement with one, two, three, etc., other improvements. The invention is thus robust in its applications and includes many different embodiments that will be apparent to the ordinarily skilled artisan from the present disclosure.

### BRIEF DESCRIPTION OF THE DRAWINGS/FIGURES

Figure 1 shows GamS-mediated inhibition of RecBCD activity.
Figure 2 shows GamS-mediated inhibition of nuclease activity in cell extracts.
Figure 3 shows that IVTT reactions in the presence of GamS yield increased protein synthesis.
Figure 4 shows consistency over time of increased protein synthesis from RNase E mutant strain extracts.
Figure 5 shows enhanced protein synthesis in IVTT reactions in the presence of acetyl phosphate.
Figure 6 shows time dependent synthesis of protein in the presence and absence of acetyl phosphate.

### DETAILED DESCRIPTION OF THE INVENTION

The present invention relates to *in vitro* synthesis of proteins and nucleic acids. The invention includes synthesis systems, methods and kits embodying one or more of the features of the present invention. The synthesis system of the present invention includes the necessary components to synthesize nucleic acids from nucleic acid templates and proteins from nucleic acid templates. The *in vitro* synthesis system of the invention provides efficient synthesis outside the confines of a cell. The methods of the present invention are useful for making systems or compositions of the present invention and for using the systems of the present invention to produce product molecules of interest. The kits of the present invention allow or facilitate the practice of the *in vitro* synthesis systems of the present invention.

The general system includes a nucleic acid template that encodes for a desired (nucleic acid (e.g. RNA or mRNA) and/or protein. The nucleic acid template can be any template, e.g., DNA, RNA, especially mRNA, and can be in any form (e.g., linear, circular, supercoiled, single stranded, double stranded, etc.). Such templates are selected for their ability to guide production of the desired protein or nucleic acid molecules. The desired protein can be any polymer of amino acids encodable by a nucleic acid template to produce a polypeptide molecule. The protein can be further processed coincident with or after synthesis. When desired, the system can be altered as known in the art such that codons will encode for a different amino acid than is normal, including unconventional or unnatural amino acids (including detectably labeled amino acids).

In this application, unless otherwise indicated, terms have their usual meaning in the context of the art of protein or nucleic acid synthesis from genetic coding material. For example:

Translation is the synthesis of a polypeptide, e.g., protein, from an mRNA template.

A nuclease is a compound with enzymatic activity resulting in the hydrolysis of polynucleotides or polynucleic acids. As a rule, the phosphodiester bonds of both DNA and RNA are resistant to hydrolysis. Biological systems have compensated by producing many nucleases that have the ability to accelerate hydrolysis of these bonds. There are various ways of classifying nucleases. For example, an endonuclease is a nuclease that cleaves its nucleic acid substrate at internal sites in the nucleotide sequence. An exonuclease is a nuclease that cleaves nucleotides sequentially from free ends of its nucleic acid substrate. Some nucleases have both endo and exo activity.

Nucleases have various functions relating to their specificities, such as, DNA repair activity, 3' to 5' or 5' to 3' specificity, double stranded or single stranded DNA or RNA:DNA specificity, etc. Nucleases can produce a 3'-terminal α,β-unsaturated aldehyde and 5' terminal phosphate (AP lyases); a 3'-hydroxyl nucleotide and 5'-terminal phosphate (class II endonuclease); or a 5'-hydroxyl and 3'-phosphate (class III endonuclease). Nuclease may preferentially process one or more types of polynucleotide, for example, single stranded DNA, double stranded DNA, mRNA, oligoRNA, ribosomal RNA, etc. Restriction nucleases are nucleases that cleave at sites with specific nucleotide sequences.

Additional properties and types of nucleases are known in the art as described in the many publications relating to nucleases that are available. Nucleases, Second Edition, Linn et al, eds. Cold Spring Harbor Laboratory Press (1993) is specifically referred to and hereby incorporated in its entirety by reference. One feature of the present invention relates to removing, preventing or inhibiting any one or more of the nuclease functions or to using cell extracts from cells having one or more nuclease or other activity deleterious or damaging to the nucleic acid template or other substrate reduced, substantially reduced or eliminated by, for example, modifying or mutating one or more genes encoding such activities.

A DNase cleaves DNA. DNA is a nucleic acid that is a common molecular basis of heredity. Common forms of DNA are single stranded and double stranded. Double stranded DNA is constructed of a double helix held together by hydrogen bonds between purine and pyrimidine bases which project inward from two chains containing alternate links of deoxyribose and phosphate. The present invention contemplates using any form of DNA, including cDNA, recombinant DNA, isolated DNA and synthetic DNA.

An RNase cleaves RNA. RNA is a nucleic acid that contains ribose and all four major bases as found in DNA, except instead of thymine, uracil is present as a structural component. RNAs are associated with the control of cellular chemical activities. Messenger RNA (mRNA) is an RNA that serves as a template for protein synthesis. RNA that encodes the protein of interest can be added to the system or can be produced from DNA present or added to the system.

"Nucleotide" refers to a base-sugar-phosphate combination. Nucleotides are monomeric units of a nucleic acids such as DNA and RNA. Nucleotides are incorporated into nucleic acids by polymerases. The term nucleotide includes, for example, ribonucleoside triphosphates such as ATP, CTP, ITP, UTP GTP, TTP or derivatives thereof and deoxyribonucleoside triphosphates such as dATP, dCTP, dITP, dUTP, dGTP, dTTP or derivatives thereof. Such derivatives include any monomer that can be incorporated into a polynucleic acid molecule, for example, [αS]dATP, 7-deaza-dGTP and 7-deaza-dATP. The term nucleotide as used herein also refers to dideoxyribonucleoside triphosphates (ddNTPs) and their derivatives. Illustrative examples of dideoxyribonucleoside triphosphates include, but are not limited to, ddATP, ddCTP, ddGTP, ddITP, and ddTTP. According to the present invention, a "nucleotide" may be unlabeled or may be detectably labeled by well known techniques. Detectable labels include, for example, different isotopes such as radioactive isotopes, fluorescent labels, chemiluminescent labels, bioluminescent labels and enzyme labels.

In the present context, the phrase, *in vitro,* refers to systems outside a cell or organism and may be sometimes be referred to cell free system. *In vivo* systems relate to essentially intact cells whether in suspension or attached to or in contact with other cells or a solid. *In vitro* systems have an advantage of being more manipulatable. Delivering components to a cell interior is not a concern; manipulations incompatible with continued cell function are also possible. However, *in vitro* systems involve disrupted cells or the use of various components to provide the desired function and thus spatial relationships of the cell are lost. When an *in vitro* system is prepared, components, possibly critical to the desired activity can be lost with discarded cell debris. Thus *in vitro* systems are more manipulatable and can function differently from *in vivo* systems.

A nucleic acid template is a polynucleic acid that serves to direct synthesis of another nucleic acid template or of a protein. The template is a molecule composed of numerous nucleotide subunits, but can vary in length and in the type of nucleotide subunits. DNA and RNA, e.g., mRNA, are species of nucleic acids that can be used as templates for protein and nucleic acid synthesis. A DNA template is transcribed to form an RNA template complementary to all or a portion of said template. An RNA template is translated to produce a protein or peptide encoded by all or a portion of the template. Thus, the template in a synthesis reaction is one or more species of nucleic acid that codes directly or indirectly for desired protein(s).

A protein is a molecule of polymerized amino acids (or derivatives thereof) bonded to one another through peptide bonds. In the present context, a polypeptide is a class of proteins. Proteins produced in accordance with the present can be assayed by any method known in the art. For example, assays specific to the produced protein or more general assays, such as radioactive assays, e.g., using ³⁵S-Met, can be used.

An energy source is a chemical substrate that can be enzymatically processed to provide energy to achieve desired chemical reactions. Energy sources that allow release of energy for synthesis by cleavage of high energy phosphate bonds such as those found in nucleoside triphosphates, e.g., ATP, are commonly used.

Other energy sources, for example sources that can form high energy phosphate bonds can also power the synthesis process. Exemplary energy sources for use in *in vitro* synthesis are glucose, phosphoenolpyruvate (PEP), carbamoyl phosphate, acetyl phosphate, creatine phosphate, phosphopyruvate, glyceraldehyde-3-phosphate, pyruvate and glucose-6-phosphate. Any source convertible to high energy phosphate bonds is especially suitable.

For example, pyruvate kinase catalyzes a reaction of PEP and ADP to form pyruvate and ATP. ATP can be reversibly converted to triphosphates of the other ribonucleosides. Thus ATP, GTP, etc. can normally be considered as equivalent energy sources for supporting protein synthesis. A protein synthesis system of the present invention can include one or more such energy source, preferably two, three, even four or more different energy sources that can generate or regenerate ATP.

An extract is a cell lysate or exudate. The cell can be any cell that can be grown for preparing an extract. Both prokaryotic cells and eukaryotic cells can be used for protein and/or nucleic acid synthesis according to the invention. Prokaryotic systems benefit from simultaneous or "coupled" transcription and translation. Eukaryotic systems are also popular. See e.g., Pelham et al, European Journal of Biochemistry, 67: 247, (1976). The ordinarily skilled artisan will appreciate that different aspects of the invention will be more or less advantageous depending on the type of cell or system, for example eukaryotic or prokaryotic systems, or the specific cell or cell line.

Preferably, the extract is processed to remove cellular debris. Centrifugation is a common method for removing such solid material. Filtration, chromatography, or any other separation or purification procedures may be used to produce a desired extract. The extract preferably includes all necessary components for synthesis that are not otherwise provided in the system. The extract can be concentrated using one or more of the many tools of the art. Enzymes and other components present in the extract to provide energy and other components for the synthesis reaction can originate in the extracted cell or can be added during the production of the extract. "Cell extract" also includes a mixture of components crafted to imitate a cell lysate or exudate with respect to the components necessary or desired for protein or nucleic acid synthesis. A cell extract thus can be a mixture of components to imitate or improve upon a cell lysate or exudate in protein synthesis reactions and/or to provide components used for synthesis from a nucleic acid template. Such mixture, as will be recognized by one of ordinary skill in the art, can be produced by obtaining a partial extract or fraction thereof and/or by mixing any number of individual components.

A gene is the fundamental physical and functional unit of heredity. Genes encode specific functional products, e.g., a protein or RNA. Genes include coding and noncoding regions. An alteration in the location of the gene or any of its coding or noncoding regions can impact function. The functional effect of a gene can be modified by altering the gene or by altering a factor controlling activity or function of the gene.

In one aspect, a gene is mutated or modified (for example, by point mutation, deletion mutation, insertion mutation, etc.) to reduce, substantially reduce or eliminate the activity of the encoded product or protein of the gene. In another aspect, the product or protein encoded by the modified gene may not be produced or if produced will have a reduced, substantially reduced or eliminated activity. To determine the relative activity, the activity from the product of the mutated gene (or cell containing it) can be compared to the activity of the product or protein encoded by the unmodified gene (or cell containing it). A mutated gene can be mutated to the extent that the activity of its encoded product or protein is no longer detectable in the cell. A deleted gene is one species of mutated gene.

A phosphatase is an enzyme that promotes or accelerates the hydrolysis of organic esters of phosphoric acid, e.g., promotes or accelerates the hydrolysis of ATP to ADP. An alkaline phosphatase is active in an alkaline pH medium. An acid phosphatase is active in an acid pH medium. Many phosphatase enzymes have one or more additional activities.

An inhibitor is a substance that reduces, substantially reduces or eliminates the activity of another compound/protein/molecule/substance, e.g., an enzyme. In the context of the present invention for maintaining nucleic acid templates, the most important inhibitors are nuclease inhibitors. Inhibitors can be, for example, proteins, metals or compounds, etc., or can be a condition (e.g., pH, ionic strength, temperature, etc.) that reduce, substantially reduce or eliminate activity of the enzyme to be inhibited. Phosphatase inhibitors and polymerase inhibitors can also be advantageously used in in *vitro* synthesis of the invention.

A polymerase is an enzyme that catalyses synthesis of nucleic acids using a preexisting nucleic acid template. When the template is a DNA template, an RNA molecule must be transcribed before protein can be synthesized. Enzymes having nucleic acid polymerase activity suitable for use in the present methods include any polymerase that is active in the chosen system with the chosen template to synthesize protein. The extract of a cell will usually contain a suitable polymerase, such as RNA polymerase II, SP6 RNA polymerase, T3 RNA polymerase, T7 RNA polymerase, RNA polymerase III and generally phage derived RNA polymerases. These and other polymerases are known in the art and can be readily assessed by the skilled artisan by searching one or more of the public or private databases. Suitable polymerase can also be supplemented in the system. When RNA is to be synthesized from a DNA template, a polymerase active on the DNA molecule of interest should be used. RNA polymerases and transcription factors useful in the invention are well known in the art and will be readily recognized by those skilled in the art.

RecBCD is an enzyme that has both single and double stranded exonuclease activity, single stranded endonuclease activity and helicase activity. The complex (and RecB in isolation) demonstrates ATPase activity. RecBCD is thus a nuclease and also a functional phosphatase.

To provide energy for the synthesis reaction, the system preferably includes added energy sources, such as glucose, pyruvate, phosphoenolpyruvate (PEP), carbamoyl phosphate, acetyl phosphate, creatine phosphate, phosphopyruvate, glyceraldehyde-3-phosphate and glucose-6-phosphate, that can generate or regenerate high energy triphosphate compounds such as ATP, GTP, etc. According to the invention, one or more (preferably two or more, three or more, etc.) of such energy sources may be used and in any combination or order of addition.

When sufficient energy is not initially present in the synthesis system, an additional source of energy is preferably supplemented. The supplement can be delivered continuously or can be delivered in one or more discreet supplements. One feature of the present invention includes addition of at least two (or three or more, four or more, five or more, six or more, etc.) energy sources to provide the energy for the synthesis reactions of the invention. Often the energy source(s), especially added energy source(s), will not themselves contain a high energy phosphate bond. Preferably they will not be triphosphate compounds, more specifically nucleotide triphosphates.

The energy source can be present in any amount that is suitable for the desired synthesis. For example, the chemical energy source can be added to achieve a concentration of from 10-100mM. About 15, 20, 25, 30, 50, 60, 70, 80 or 90 mM may also be target concentrations. The precise concentration will vary as synthesis consumes energy and the energy is replenished from these sources. The concentration may be controlled within various ranges, for example about 10-100 mM, 15-90 mM, 20-80 mM, 30-60 mM, etc. Any target concentration can be used as an approximate boundary for the desired range of concentration of energy source. Energy sources can also be added or supplemented during the *in vitro* synthesis reaction.

When two or more energy sources are used, each source can independently be targeted to be one of these target concentrations.

When multiple energy sources are included in the system, synthesis (especially protein synthesis) is found to be accelerated and prolonged in time, so that protein and/or nucleic acid products are more efficiently produced by the synthesis system. For example, when phosphoenol pyruvate (PEP) and acetyl phosphate are used as added energy sources, the amount of protein synthesized can be more than doubled as compared to when only acetyl phosphate is added. Thus, the present invention includes an *in vitro* synthesis system that comprises at least one, and preferably at least two or more different energy sources that provide high energy phosphate bonds for the synthesis reactions.

The synthesis systems of the invention are based on one or more cell extracts. Cells possess the necessary components to synthesize proteins and/or nucleic acids. Extracts of cells thus are a readily obtainable source of synthesis components. Components processed from cells or components processed or synthesized from other sources can be included in the extract.

Maintenance of the template is necessary to maximize the duration of the synthesis process. Thus the synthesis system of the present invention can include components that maintain the template. The template can be maintained by preventing enzymatic, chemical or other degradation of the template.

The synthesis system of the invention therefore can include modifications to the extract to improve product synthesis. When the extract contains enzymes whose activities compromise protein and/or nucleic acid production, inhibition of these enzymes will result in more efficient synthesis by the system. Thus, *in vitro* synthesis systems comprising inhibitors of at least one enzyme are embodiments of the present invention. Nuclease and phosphatase inhibitors are advantageously used to increase protein and/or nucleic acid synthesis efficiency. Inhibition of enzymes that unnecessarily consume compounds used in the synthesis reaction can also improve synthesis efficiency. Depending on the specific enzymes present in the extract, for example, one or more of the many known nuclease, polymerase or phosphatase inhibitors can be selected and advantageously used to improve synthesis efficiency.

However, it may not be desirable to prevent or inhibit the activities of all nucleases. For example, RNase I and RNase I* preferentially degrade short RNA oligonucleotides as compared to full length RNAs. In some circumstances this activity can prove helpful. Cells use this enzyme to degrade RNA that is no longer needed. For example, mononucleotides made available by RNase I would be available as substrates for transcribing DNA. Alternatively, blocking RNase I may be insufficient by itself to maintain the RNA for translation during the protein synthesis process. For example, other enzymes may be more active in initial degradation of mRNA.

To maintain the template, cells that are used to produce the extract can be selected for reduction, substantial reduction or elimination of activities of detrimental enzymes or for enzymes with modified activity. Thus, *in vitro* synthesis systems comprising extracts of cells having altered activity (for example by modifying or mutating one or more genes) are embodiments of the present invention. Cells with modified nuclease or phosphatase activity (e.g., with at least one mutated phosphatase or nuclease gene or combinations thereof) are especially advantageously used to increase synthesis efficiency.

The invention also embodies an *in vitro* synthesis system where one or more polymerases are modified (or polymerase activity is modulated). For one aspect, polymerase activity is reduced or substantially reduced or inhibited (for example by mutating a polymerase gene or its regulatory elements) to maintain the template. For example, RNAs bound to ribosomes are protected from RNase E. However, when the polymerase activity is too great, then the RNase E may degrade the RNA before a ribosome can bind. Thus, the template can be maintained by inhibiting RNA polymerase or by using cells with reduced polymerase activity. A cell with an RNA polymerase modified so that the polymerization speed is modulated to improve ribosome binding may be used according to the invention to produce a protein synthesis system where RNA is more efficiently utilized. Also, DNA polymerases may not be needed in some systems (e.g., translation systems) and thus the invention relates to inhibition, reduction, substantial reduction or elimination of one or more polymerases such as a DNA polymerase, for example to prevent such DNA polymerase from utilizing reaction components (e.g., prevent unnecessary consumption of energy.

Alternatively, the cell for producing the extract can be grown under conditions such that a product or protein or activity is reduced or modulated or eliminated (e.g., to reduce expression of a protein interest). Such growth conditions are considered a type of modulation or inhibition according to the invention. The resulting extracts will be similar to extracts obtained from cells where a gene is mutated or modified.

The present invention also includes methods for production of protein and/or nucleic acid molecules in an *in vitro* synthesis system. The methods of the present invention include producing such products with *in vitro* systems having at least one, preferably at least two or three or more energy sources that may be added, either continuously or by one or more discreet additions. The methods of the present invention also include producing proteins and/or nucleic acids with *in vitro* systems modified to maintain the nucleotide template. For example: nucleases, phosphatases, or polymerases can be inhibited by chemical or physical conditions; and/or extracts from cells deficient or mutated or modulated in one or more enzyme, such as nuclease, phosphatase or polymerase can be used a basis for the system.

Mutated or modified cells can be made as indicated in the examples and/or can be made according to methods known in the art. In the context of the present invention a modified cell is a cell with one or more mutated or modified genes or modified or modulated in such a way to reduce, substantially reduce, eliminate, inhibit or modulate certain activity or activities of interest. A mutated gene includes a gene that is not transcribed or translated into the fully functional gene product associated with the wild type gene. A mutation can be any mutation that does not result in full function of the wild type gene product. For example, the mutation can be a point mutation, a complete or partial deletion of the gene, a complete or partial substitution of the gene and/or one or more insertions in a gene.

The extract can be made from any suitable cells. Suitable cells are those that have components for protein and/or nucleic acid synthesis, optionally produced under selected growth conditions or with modification(s) and/or mutation(s) that inactivate or reduce or substantially reduce unwanted properties detrimental to *in vitro* synthesis. Host cells that may be used according to the invention include, but are not limited to, bacterial cells, fungal and yeast cells, plant cells and animal cells. Preferred bacterial host cells include, for example, gram positive and gram negative bacteria, and especially *Escherichia* spp. cells (particularly *E. coli* cells and most particularly E. *coli* strains DH10B, Stbl2, DH5α, DB3, DB3.1 (preferably E. *coli* LIBRARY EFFICIENCY^{®} DB3.1^{™} Competent Cells; Invitrogen Corp., Life Technologies Division, Rockville, MD), DB4 and DB5 (*see* U.S. Application No. 09/518,188, filed on March 2, 2000, and U.S. Provisional Application No. 60/122,392, filed on March 2, 1999, the disclosures of which are incorporated by reference herein in their entireties), BL21, HB101, INV110, INVαF', MC1061/P3, PIR1, PIR2, TOP10, TOP10F', TOP10/P3, etc., *Bacillus* spp. cells (particularly *B. subtilis* and *B. megaterium* cells), *Aspergillus* spp. cells, *Streptomyces* spp. cells, *Erwinia* spp. cells, *Klebsiella* spp. cells, *Serratia* spp. cells (particularly S. *marcessans* cells), *Pseudomonas* spp. cells (particularly *P. aeruginosa* cells), and *Salmonella* spp. cells (particularly S. *typhimurium* and *S*. *typhi* cells). Preferred animal host cells include insect cells (especially, *Drosophila melanogaster* cells and more especially S2 cells, *Spodoptera frugiperda* Sf9 and Sf21 cells and *Trichoplusa* High-Five cells), nematode cells (particularly C. *elegans* cells), avian cells, amphibian cells (particularly *Xenopus laevis* cells), reptilian cells, and mammalian cells (most particularly NIH3T3, CHO, COS, C127, VERO, BHK, HeLa, 293, Per-C6, Bowes melanoma, and human, rabbit, mouse, rat, hamster, pig, bovine and gerbil cells generally). Preferred yeast host cells include *Saccharomyces cerevisiae* cells and *Pichia pastoris* cells. These and other suitable host cells are available commercially, for example, from Invitrogen Corp., Life Technologies Division (Rockville, Maryland), American Type Culture Collection (Manassas, Virginia), and Agricultural Research Culture Collection (NRRL; Peoria, Illinois), the catalogues of each being incorporated in their entireties by reference. Plant cells are exemplified by protoplasts, tobacco, potato and other tuberous plants, grasses including maize, cotton and other fibrous plants, annuals and perennials, monocots and dicots, and especially plant cells that can be transformed and/or grown in culture.

The cell extract can be supplemented to provide components not present or not present in sufficient quantities after extraction.

The extract can be prepared by any method used in the art that maintains the integrity of the transcription/translation system or if the process damages one or more component necessary for any stage of transcription/translation, the damaged component can be replaced or substituted for after the extract preparation.

Extracts were prepared according to the method of Zubay (1973) referenced above. The ordinarily skilled artisan will recognize that many modifications to the extraction process are possible within the scope of the present invention.

Unless otherwise specified, the incubation reaction contained 57 mM Hepes/KOH pH 8.2, 230 mM K-glutamate, 1.2 mM ATP, 0.85 mM each of GTP, UTP, CTP, 30 mM PEP, 1.7 mM DTT, 12 mM Mg(OAc)₂, 0.17 mg/ml *E.coli* total RNA mixture, 34 µg/ml folinic acid, 66 µg/ml T7 RNA polymerase, 1.25 mM each of amino acids, 14 mg/ml extract, 3 µl of ³⁵S-Met (15 µCi/µl) and various amount of DNA template.

The skilled artisan will recognize that concentrations of the various components of the incubation medium can be adjusted as is known in the art while still maintaining the synthetic function. For example, the pH can range from about 5.6 to about 8.8 or more preferably about 6.1 to 8.5 or even about 7.2 to 8.4 depending on the product to be synthesized. Likewise the concentration of K-glutamate can be easily varied between about 80 mM and 320 mM or more preferably about 120 to 280 mM or even about 180 to 250 mM; the concentration of ATP can be varied from about 0.1 to 3.0 mM or more preferably about 0.3 to 2.0 mM or even about 0.8 to 1.5 mM; the concentrations of GTP, UTP CTP and TTP can vary from about 0 or 0.1 to 2 mM or more preferably about 0.4 to 1.2 mM or even about 0.7 to 1.0 mM; PEP can vary from about 10 to 60 mM or more preferably about 20 to 50 mM or even about 25 to 40 mM; DTT can vary from about 0.5 to 3.0 mM or more preferably about 1.0 to 2.5 mM or even about 1.5 to 2.0 mM; magnesium can vary from about 7.5 to 20 mM or more preferably about 10 to 15 mM or even about 11.5 to 14 mM; total RNA mixture can vary from about 0.1 to 0.5 mg/ml or more preferably about 0.12 to 0.3 mg/ml or even 0.15 to 0.2 mg/ml; folinic acid can vary from about 15 to 50 µg/ml or more preferably about 25 to 40 µg/ml or even about 30 to 35 µg/ml;polymerase can vary from about 0 or 1.0 to 300 µg/ml or more preferably about 20 to 200 µg/ml or even about 30 to 150 µg/ml, about 40 to 120 µg/ml, about 50 to 100 µg/ml or about 60 to 75 µg/ml; amino acids can vary independently from about 0.4 to 5 mM or more preferably about 0.5 to 2.0 mM or even 0.8 to 1.5 mM; extract can vary from about 2 to 40 mg protein/ml or more preferably about 5 to 25 mg protein/ml or even about 10 to 18 mg protein/ml; and a synthesis marker, if desired, (³⁵S-Met in the example described above) can be selected as desired and used in any amount detectable that does not unduly compromise synthesis. The skilled artisan will readily recognize that many of the components have known substitutes or equivalents that might be used either in the same concentration or in a concentration that produces a qualitatively and/or quantitatively similar effect.

### EXAMPLE 1

### In vitro cell-free protein synthesis using mutant E.coli

A mutant derivative of BL21 was used for cell-free protein synthesis in the IVTT system of the present invention. The starting BL21 strain was devoid of OmpT and Ion proteases (Studier, Methods in Enzymology, 185: 60-89, (1990)). The following genes were mutated: RNase I, the carboxy-terminus deletion of RNase E and endonuclease I by techniques known in the art. These mutations resulted in stabilizing or maintaining the DNA template and mRNA as well as stabilizing the proteins being synthesized. It has been reported that growth of *E.coli* cells in high phosphate containing media represses the synthesis of alkaline phosphatase (Malamy and Horecker, Biochemistry, 3: 1893-1897). Growing cells in high phosphate medium was therefore attempted to produce cells with low phosphatase activity. Additional mutations of other nucleases or other detrimental genes have can also be made to further enhancement (e.g., see below, Example 6)). The extracts made of from mutated *E.coli* grown in phosphate containing media enhance the production of proteins.

### EXAMPLE 2

### Cloning and Expression of lambda Gam

Instead of mutating the RecBCD, a novel method for inactivating the RecBCD nuclease has been found. The novel method involves incorporating lambda recombinase protein, Gam, in the cell-free extract.

Gam has been shown to inhibit *E.coli* RecBCD, *in vivo,* and has helped in homologous recombination using linear DNA (Yu et al, PNAS, 97: 5978-5983, (2000); Datsenko and Warner, PNAS, 97: 6640-6645, (2000)). In both cases, the authors demonstrated that, in the presence of lambda Gam, a linear DNA can be protected and the lambda Exo and Beta promote highly efficient homologous recombination. Gam was used here in the *E.coli* cell-free extract to attempt to protect linear DNA and thus potentially enhance protein and/or nucleic acid synthesis.

### Multiple Gam Isoforms

In the literature, there are several references to controversy over the size of the *gam* gene product (Murphy, J. Bact., 173: 5808-21, (1991); Friedman and Hays, Gene, 43: 255-63, (1986)). At least one researcher has reported that two separate forms of Gam were detected on SDS-PAGE: one at an apparent molecular weight of 17 kD, and one at about 12 kD. It was proposed, and later demonstrated, that the smaller protein was indeed a product of the *gam* gene, but started at an internal methionine rather than the previously predicted start site upstream. This internal start site had a good Shine-Dalgarno sequence (AGGAGTTCAGCC) (SEQ ID NO:1), whereas the originally mapped start site had no detectable translation start sequence.

In addition, one report (Murphy, 1991) suggested that this short form of Gam, called GamS, had all of the RecBCD inhibitory activities of the in *vivo gam* gene product. For these reasons, we decided to clone the short form of Gam and try to express protein from it.

Primers DE230 (SEQ ID NO:2) (5'-GGGAGGCCATGGATATTAATACTGAAACTGAG) and DE231 (SEQ ID NO:3) (5'-GGGAGGAGATCTTTATACCTCTGAATCAATATCAACC) were used to PCR amplify the lambda *gam* gene from pKD46. The PCR fragment was digested with NcoI and Bgl II sites, and introduced into pBAD-HisA cut with the same enzymes. The construct produced, pLDE129, contained Gam under the control of the pBAD promoter using an engineered optimal Shine-Dalgarno sequence (AGGAGGAATTAACC) (SEQ ID NO:4).

GamS was cloned as described above for Gam, using primers DE255 (5'-GGGAGGCCATGGGAAACGCTTATTACATTCAGGATCGTCTTG) (SEQ ID NO:5) and DE231 (SEQ ID NO:3) (5'-GGGAGGAGATCTTTATACCTCTGAATCAATATCAACC). The final construct, pLDE136, was introduced into DH10B (Life Technologies) and tested for expression. These cells expressed significant levels of a protein with an apparent molecular weight of 12 kD, exactly as expected for GamS. Small-scale extracts were made as described previously, and in the case of GamS, all of the protein appeared in the soluble fraction.

pLDE129 was introduced into E. *coli* DH10B, grown to an OD600 of 0.6 at 37°C, and induced with 0.2% arabinose. After 3 hours of induction, 0.05 OD of cells were removed and subject to SDS-PAGE analysis on a 4-12% NuPage gel alongside 0.05 OD of uninduced cells. Cells expressing Gam produced a significant protein band at an apparent molecular weight of 17 kD, corresponding nicely to the predicted size of Gam (16.3 kD). A 2 ml induced culture of Gam was centrifuged, and the cells were frozen at -80°C, thawed, and resuspended in 0.4 ml extract buffer (50 mM Na-Phosphate, pH 7.0, 10% glycerol). The resuspended cells were sonicated twice for 30 seconds using a 1/8 inch probe at 50% power for 30 seconds, NaCl was added to a final concentration of 0.5M, and the extract was clarified by centrifugation. Clarified extract and insoluble pellet fractions were electrophoresed and compared to whole cell extracts. Though Gam was present in significant amounts in the whole cell extracts, the soluble extract contained no detectable Gam protein. Instead, nearly all of the protein was in the insoluble pellet fraction.

### EXAMPLE 3

### Activity Assay for Gam-mediated RecBCD inhibition

In order to assay Gam *in vitro,* we have developed a radioactive assay. Briefly, a double-stranded uniformly labeled linear DNA is used as the substrate for RecBCD activity utilizing purified RecBCD protein (Plasmid-Safe ExoV, Epicentre). Gam protein is preincubated with RecBCD and ATP, and then added to the DNA. After an incubation period, the mixture is bound to GFB filters, washed to remove small DNA fragments, and the counts remaining on the filter are determined. By comparing to the number of counts remaining using RecBCD without preincubation with Gam, the percent inhibition can be determined.

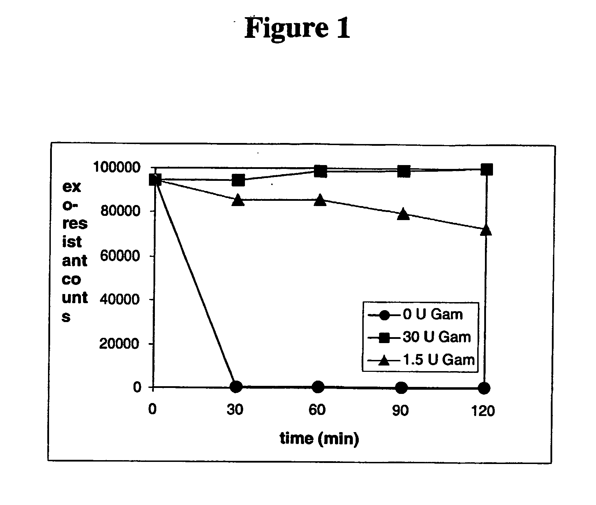

As shown in Figure 1, 0, 1.5, or 30 units of GamS were incubated with 1 unit of purified RecBCD (Plasmid-Safe, Epicentre) in 100 µl 1x Plasmid-Safe buffer and 10 mM ATP for 10 minutes at 37°C. 50 fmol of internally ³²P-labelled exonuclease substrate was added, and the reaction was continued at 37°C for 30, 60, 90, or 120 minutes. Reactions were stopped by the addition of 100 µl filter binding buffer (6M guanidine, 100 mM MES) and kept on ice until all reactions were complete. Reaction mixtures (150 µl) were spotted onto Millipore GF/B filters, washed 3x with 200 µl 80% ethanol, dried, placed in 4 ml Scintisafe-F, and counted for 1 minute. GamS inhibits degradation of the substrate in a concentration dependent manner.

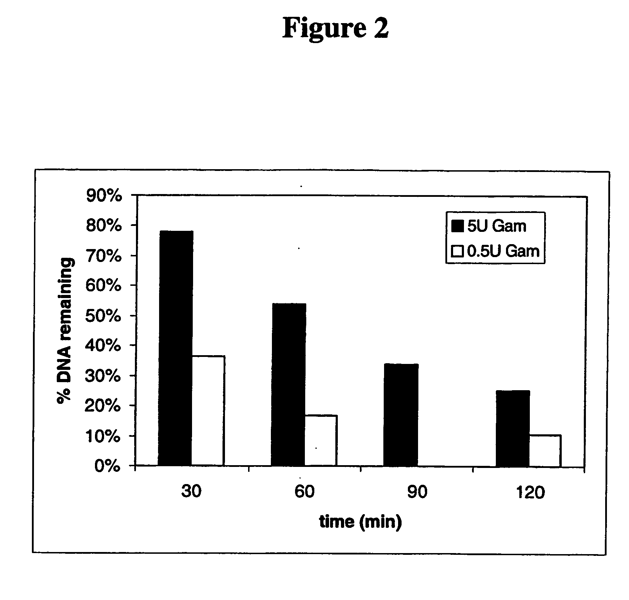

As shown in Figure 2, 0.5 or 5 units of GamS were incubated with 1 µl (approximately 40 µg) of cell-free extract in 100 µl 1x Plasmid-Safe buffer and 10 mM ATP for 10 minutes at 37°C. Exonuclease assays were carried out as described in the legend to Figure 1. Percent DNA remaining was determined by dividing the counts bound in each assay by the counts bound in controls lacking cell-free extract.

### EXAMPLE 4

### Purification of GamS

An overnight culture of pLDE136 was diluted into 15 ml LB-Amp in a 50-ml flask, and grown to an OD of 0.6 at 37°C. Arabinose was added to a final concentration of 0.2%, and the cells were grown for an additional 3 hours before harvesting. The pellet was frozen, thawed, resuspended in 500 µl extract buffer (50 mM Tris-Cl, pH 8.0, 0.1 mM EDTA, 10% glycerol, 500 mM NaCl), sonicated twice for 30 seconds using a 1/8 inch probe at 50% power, and clarified by centrifugation. The sample was diluted to 100 mM NaCl prior to chromatography. A 1 ml Hitrap-MonoQ column (Pharmacia) was equilibrated with 10 ml of buffer B (50 mM Tris-Cl, pH 8.0, 0.1 mM EDTA, 10% glycerol, 1M NaCl, 1 mM DTT) and then with 10 ml of buffer A (50 mM Tris-Cl, pH 8.0, 0.1 mM EDTA, 10% glycerol, 1 mM DTT), before being washed extensively with 10% buffer B. The extract was loaded at 10% buffer B at a flow rate of 0.5 ml/min, washed with 10% buffer B at 0.5 ml/min for 15 CV, and eluted with a 20 CV gradient of 10%-70% buffer B at 0.25 ml/min. Fractions were collected every 0.5 ml. GamS eluted in 3 main fractions at a salt concentration of approximately 400 mM. These fractions were analyzed on SDS-PAGE, and pooled into a single MonoQ pool. By Coommassie staining, this pool was estimated to be between 70-80% pure.

### EXAMPLE 5

### Assay of partially purified GamS

The GamS MonoQ pool was assayed along with the Gam extracts to determine the amount of activity present. The MonoQ pool had 25-30 U/µl of activity, and was 194 ng/µl in protein concentration, giving a specific activity value between 160-200 U/µg of actual GamS protein (assuming 75-80% purity). Based on activity assays of the extract, very little activity was lost over the MonoQ column, and any losses were most likely due to conservative pooling. Aliquots of full-length Gam extracts showed a small but detectable level of activity-upon checking the SDS-PAGE gels more carefully, a small amount of soluble full-length Gam is found to be present. This soluble amount probably represents any activity observed for full length Gam.

### EXAMPLE 6

### Stabilization of linear DNA against purified RecBCD and in E.coli cell-free extract in the presence of Gam

Analysis of *E.coli* cell-free extracts using the radioactive exonuclease assay showed that there are significant levels of RecBCD-like exonuclease activity in the extracts (approximately 0.4 units/µl extract). Addition of purified GamS to the extract protected most of the substrate from degradation. However, 20% of the substrate was still degraded even at optimal levels of GamS. This suggests that the extract contains at least some other nucleases which are not inhibited by the presence of GamS. This result was further confirmed by experiments performed at longer incubation times. We have shown that linear DNA (PCR fragment) can be completely protected against purified RecBCD in the presence of GamS for incubation times up to 4 hours. (Figure 1). However, in experiments involving GamS and crude extracts, lengthening the incubation time leads to the degradation of more substrate, independent of the levels of GamS added. After 30 minutes, 80% of the substrate remains; after 2 hours of incubation, only 30% of the substrate remains protected (Figure 2). It is therefore likely that other *E.coli* nucleases, (such as ExoIII, ExoVIII, EndoIV and double stranded DNA specific nucleases) are also acting on the linear DNA with the result that full protection was not achieved. Therefore, mutations or inhibition of any or all of these genes are expected be useful to protect the template for longer periods of time.

### EXAMPLE 7

### Enhanced Protein production using linear DNA in the cell-free extract in the presence of Gam

Cell-free *in vitro* transcription-translation reactions (50 µl) were prepared as described above using either a supercoiled plasmid DNA template or a PCR product template (50 ng). Each template encodes the CAT gene under the control of the T7 promoter. The PCR product was made directly from the supercoiled plasmid. GamS protein (200 ng) was added to *E.coli* extract and pre-incubated for 2 minutes at room temperature before addition to reactions. Reactions were carried out at 37°C for 2 hours. To quench reactions, RNase A (5ug) was added and reactions were incubated at 37°C for 15 minutes. Reactions were spotted (5 µl) on GFC filters, washed 1X in 10% trichloroacetic acid, 2X in 5% trichloroacetic acid and 1X in methanol. Filters were dried, placed in 4ml Scintisafe-F, and counted for 1 minute. The amount (pmoles) of incorporated methionine for each sample is a measure of protein synthesis.

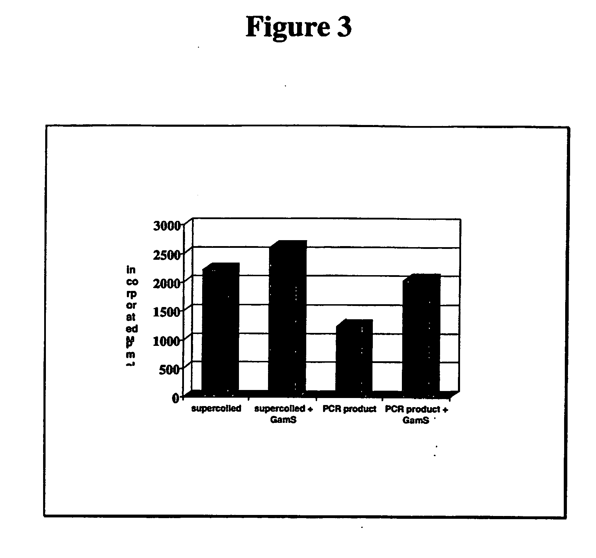

Cell-free protein synthesis was followed in the presence or absence of GamS protein. Supercoiled plasmid DNA or PCR products containing a CAT gene under the control of T7 promoter were used as substrates for protein synthesis. In the absence of Gam protein, yield from a PCR product DNA template is decreased (2-fold) as compared to the yield from a supercoiled DNA plasmid template. Addition of GamS protein to the reaction has no effect on synthesis from the supercoiled template. However, GamS restores protein production from the PCR product DNA template to a level comparable to that observed from the supercoiled DNA template (Figure 3).

### EXAMPLE 8

### Enhancement of protein synthesis in RNase E deletion mutant

As described above, stability of mRNA is an important factor in producing more protein either *in vivo* or *in vitro.* Therefore, inactivation of one or more RNase(s) might increase the half-life of mRNA, which in turn might enhance the production of proteins. In *E.coli,* transcription and translation are coordinated, and the ribosome binds newly synthesized mRNA as soon as the ribosome binding site is exposed (Stent, G.S., Proc. R. Soc., 164: 181-197, (1966); Steitz, J.A, Nature, 224: 957-964, (1969); Miller et al, Science: 169: 392-395, (1970).

The T7 promoter, one of the strongest promoters in *E.coli,* has been widely used for expressing proteins. With the T7 promoter, however, the mRNA is made approximately 8-times faster than mRNA made with native polymerase and also 8 times faster than the message is translated on the ribosome. This creates a situation where mRNA has ribosome-free portions and thus, can become the target for RNase E (Iost et al, J. Bacteriol. 174: 619-622; Makarova et al, PNAS, 92: 12250-12254, (1995)). Because of this reason, the authors suggested that the expression of protein per transcript is lower when the T7 promoter is used. One feature of the present *in vitro* protein synthesis invention is to use a modulated polymerase that preserves or maintains the nascent mRNA in the cell free system. Mutated T7 polymerases are well known in the art. See for example, Bonner et al, EMBO J., 11: 3767-3775, (1992).

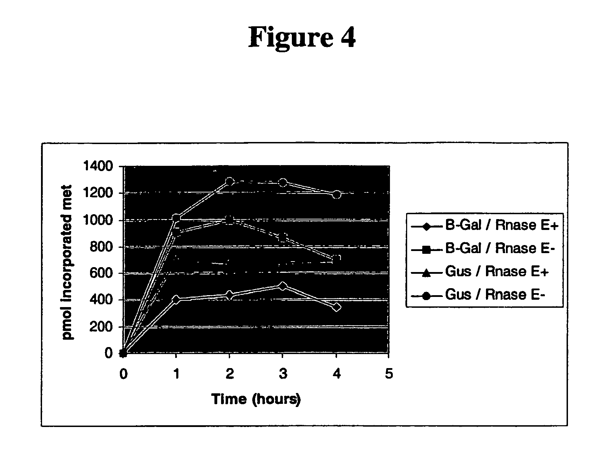

Recently, Lopez et al, (Mol. Microbiol. 33: 188-199, (1999)) reported that the protein synthesis per transcript *in vivo* could be increased if an *E.coli* strain with a mutation in RNase E is used as a host for expression. In *in vitro* transcription-translation systems, the T7 promoter is often used in place of the native *E. coli* promoter and polymerase. Therefore, we tested if cell-free extract made from an RNase E mutant of *E.coli* might also enhance the synthesis of proteins using the different promoter system. Protein expression from four different plasmid templates, each containing the T7 promoter, was assayed using cell-free extract made from both an RNase E mutant strain and a wild type strain. Our results as described below suggest that cell-free extract of RNase E mutant *E.coli* enhanced protein synthesis up to 6 -fold compared to wild type *E.coli* (Table 1 and Figure 4). It therefore appears that coordinating transcription, translation and enzymatic degradation can improve protein synthesis efficiency.

Cell-free *in vitro* transcription-translation reactions were carried out using three independent supercoiled plasmid DNA templates. These templates each encode a mammalian protein, Gus, v-Raf or tTak, under the control of the T7 promoter. Each template was tested for protein synthesis using extracts made from both the wild type (BL21-ERP) and the RNase E⁻ (BL21-Star, Invitrogen Corp.) strains. Two amounts of each DNA template were assayed (500 ng and 100 ng). The amount (pmoles) of incorporated methionine is shown for each sample as a measure of protein synthesis. Fold increase in protein synthesis when using the RNase E- strain is shown to the right.

**TABLE 1**

| RNase E Deletion Increases Protein Synthesis In IVTT Reactions | | | |
|---|---|---|---|
| ***Template*** | pmol incorporated ***Met*** | pmol incorporated ***Met*** | ***Fold Increase*** |
| | ***Rnase E +*** | **Rnase E -** | |
| *Gus* | | | |
| | 455 | 1194 | 2.6 |
| 500ng | 50 | 279 | 5.6 |
| 100ng | | | |
| **v-Raf** | | | |
| 500ng | 1700 | 2318 | 1.4 |
| 100ng | 810 | 1657 | 2.0 |
| **tTak** | | | |
| 500ng | 505 | 449 | 1.0 |
| 100ng | 25 | 144 | 5.7 |

To test whether enhanced protein synthesis from the RNase E mutant strain is consistent over time, a time course experiment was carried out. Expression of two proteins, B-Gal and Gus, was assayed after 1, 2, 3 and 4 hours. Cell-free *in vitro* transcription-translation reactions (100 µl) were prepared as described above using extract made from either the RNase E mutant strain BL21-Star (Invitrogen Corp, Carlsbad, California) or a wild type strain (BL21-ERP). Reactions contain supercoiled plasmid DNA templates (1 µg) encoding either the B-Gal or Gus gene under the control of the T7 promoter. Reactions were incubated at 37°C and aliquots (20 µl) from each reaction were removed after 1, 2, 3, and 4 hours and quenched by treatment with RNase A. Aliquots were placed on ice until all reactions were complete. Samples were processed as described above in Example 7. The amount (pmoles) of incorporated methionine for each sample is a measure of protein synthesis.

Results indicate that expression of protein is higher when using the RNase E mutant strain as compared to a wild type strain and this effect is constant over time. Furthermore, protein expression appears to be maximal after only 1 hour as no significant increase in expression was observed at later time points (Figure 4).

RNase I and RNase E are not the only RNases that degrade RNAs. A number RNases act on RNA (Linn and Deutscher: Nucleases, pages 455-468, Cold Spring Harbor Laboratory Press, (1993)). The current data show that mutation(s) or inhibition of any of these RNases could also be beneficial.

### EXAMPLE 9

### Dual-energy source for enhanced protein synthesis

The biochemical energy source in an *in vitro* protein synthesis system is derived from the hydrolysis of triphosphates the concentration of which is maintained by an energy regeneration system. Commonly used compounds are creatine phosphate for eukaryotic systems and phosphoenolpyruvate (PEP) for bacterial systems. In some cases, acetyl phosphate has been used (Ryabova et al, Anal. Biochem., 226, 184-186, (1995)). In the acetyl phosphate system, it has been shown that the level of ATP can be maintained twice as long as in a PEP system. The optimal concentration of acetyl phosphate, in terms of protein synthesis, was found to be about 25-30 mM. Addition of higher concentration, e.g., 40 mM, was found to be inhibitory. To determine the effect on protein synthesis, we titrated acetyl phosphate into IVTT reactions in the absence or presence of PEP.

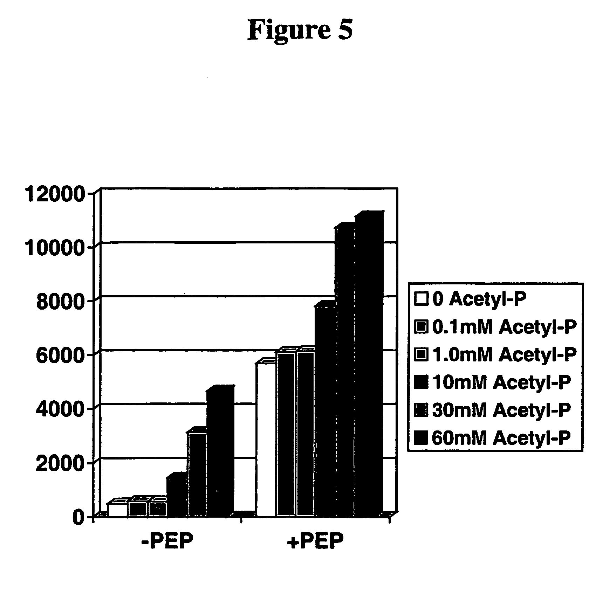

Cell-free *in vitro* transcription-translation reactions were carried out using 750 ng of supercoiled plasmid DNA encoding the CAT gene driven by the T7 promoter. Acetyl phosphate was titrated into the reactions (0.1 mM to 60 mM) in the absence or presence of 30 mM PEP. Total reaction volume was 50 µl. Reactions were incubated at 37°C for 2 hours. Reactions were quenched with RNase A and precipitated with trichloroacetic acid as described for Figure 3. In this protocol, surprisingly, no inhibition of protein synthesis is observed at least up to 60 mM of acetyl phosphate (Figure 5).

There was almost linear increase of protein synthesis as the concentration of acetyl phosphate increased. Addition of acetyl phosphate alone stimulates protein synthesis to a level that is comparable to that observed when using just PEP. Furthermore, addition of acetyl phosphate in combination with PEP increases protein production two-fold. Thus, there appears to be an especially beneficial effect on protein synthesis when adding both PEP and acetyl phosphate to IVTT reactions.

To demonstrate the effect of acetyl phosphate on protein synthesis over time, a time course experiment was carried out. CAT protein was synthesized in IVTT reactions containing 30 mM PEP and/or 60 mM acetyl phosphate. As a control, reactions were also performed with no exogenous energy source. Reactions were prepared as described above using 750 ng of the T7-CAT plasmid.

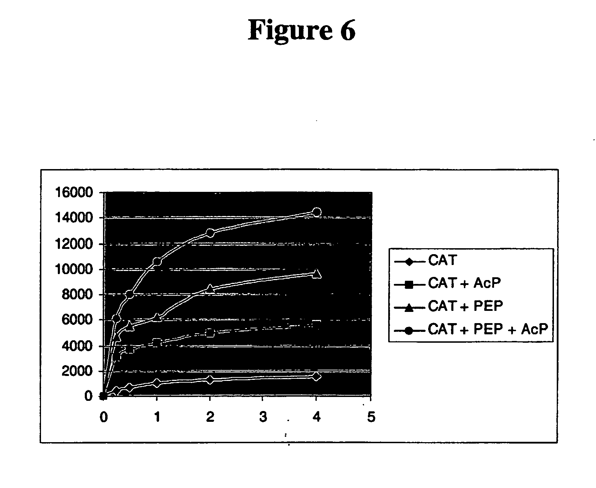

Samples were processed as described in Example 7. The amount (pmoles) of incorporated ³⁵S-methionine for each sample was used to quantify protein synthesis. As seen in Figure 6, the results demonstrate that presence of acetyl phosphate increases protein synthesis when added to reactions containing PEP. This effect was observed over the entire four hour time course of the experiment.

In view of the above description, examples and associated Figures of the present invention the skilled artisan is hereby enabled to practice the invention as described herein and to modify the present invention in accordance with the art to practice same within a wide range of varied conditions without affecting the scope of the present invention.

## Claims

1. An *in vitro* synthesis system for producing protein or nucleic acid from a nucleic acid template, comprising an extract from a bacterial cell, said cell having a mutation resulting in reduced activity of at least one nuclease, **characterised in that** said extract is modified by addition of Gam protein.

2. An *in vitro* synthesis system according to claim 1, wherein the Gam protein is soluble Gam protein.

3. The *in vitro* synthesis system according to either claim 1 or claim 2, wherein the template is a DNA template and the nuclease is a DNase.

4. An *in vitro* synthesis system according to claim 3, wherein the DNase is a DNA exonuclease.

5. An *in vitro* synthesis system according to claim 3, wherein the DNase is a DNA endonuclease.

6. An *in vitro* synthesis system according to claim 5, wherein the DNA endonuclease is endonuclease A.

7. An *in vitro* synthesis system according to either claim 1 or claim 2, wherein the nuclease is an RNase.

8. An *in vitro* synthesis system according to claim 7, wherein the RNase is an RNA exonuclease.

9. An *in vitro* synthesis system according to claim 7, wherein the RNase is an RNA endonuclease.

10. An *in vitro* synthesis system according to claim 9, wherein the endonuclease is RNase E.

11. An *in vitro* synthesis system according to claim 9, wherein the endonuclease is RNase I or RNasel*.

12. An *in vitro* synthesis system according to any preceding claim, further comprising at least one nucleic acid template selected from the group consisting of a DNA template and an RNA template.

13. An *in vitro* synthesis system according to any preceding claim, comprising at least one DNA template and wherein the *in vitro* synthesis system is an *in vitro* transcription/translation system.

14. An *in vitro* synthesis system according to any preceding claim, comprising at least two added energy sources that generate or regenerate high energy triphosphate compounds.

15. An *in vitro* synthesis system according to claim 14, comprising at least two added compounds selected from the group consisting of pyruvate, phosphoenolpyruvate (PEP), carbamoyl phosphate, acetyl phosphate, creatine phosphate, phosphopyruvate, glyceraldehyde-3-phosphate and glucose-6-phosphate.

16. An *in vitro* synthesis system according to claim 15, comprising added acetyl phosphate.

17. An *in vitro* synthesis system according to claim 16, further comprising added phosphoenolpyruvate.

18. An *in vitro* synthesis system according to claim 17, comprising about 30mM phosphoenolpyruvate and about 60mM acetyl phosphate.

19. An *in vitro* synthesis system according to any preceding claim, wherein the extract from said cell is reduced in the activity of at least one enzyme selected from the list consisting of OmpT, RNase E, alkaline phosphatase and endonuclease I.

20. An *in vitro* synthesis system according to any preceding claim, wherein the cell extract is from an *Escherichia coli* cell.

21. A composition comprising:
a. an extract from a bacterial cell, said bacterial cell having at least one mutation resulting in reduced activity of a nuclease,
b. at least two added energy sources providing chemical energy for synthesis
c. added Gam protein.

22. A composition according to claim 21, further comprising at least one nucleic acid template in the presence of at least a partial synthesis product of said template.

23. A composition according to claim 22, wherein the product is a nucleic acid product.

24. A composition according to claim 23, wherein the nucleic acid product is DNA.

25. A composition according to claim 23, wherein the nucleic acid product is RNA.

26. A kit for *in vitro* synthesis comprising:
a. an extract from a bacterial cell, said bacterial cell having at least one mutation resulting in reduced activity of a nuclease,
b. added Gam protein
c. one or more added reagents selected from the list consisting of nucleotides or nucleotide derivatives, at least one polymerase, at least one cofactor, at least one buffer or buffer salt, at least one energy source for synthesis, at least one nucleic acid template, at least one reagent to determine the efficiency of the kit.

27. A kit according to claim 26, comprising two or more energy sources for synthesis.

28. A method for producing protein or nucleic acid from a nucleic acid template in an *in vitro* system comprising: contacting said template with
a. at least one extract from a bacterial cell, said cell having a mutation resulting in reduced activity of at least one nuclease,
b. Gam protein; and
c. at least two added energy sources providing chemical energy for synthesis;
forming a mixture; and incubating said mixture under conditions sufficient to produce at least one protein encoded by said template.

29. A method according to claim 28, wherein the at least one nuclease is selected from the group consisting of RNase E, RNase I and endonuclease I.

30. A method according to either claim 28 or claim 29, wherein the Gam protein inhibitor is soluble Gam protein.

31. A method according to any of claims 28 to 30, wherein each of the at least two added energy sources generates or regenerates high energy triphosphate compounds for protein synthesis.

32. A method according to claim 31, wherein the at least two added energy sources are selected from the group consisting of pyruvate, phosphoenolpyruvate (PEP), carbamoyl phosphate, acetyl phosphate, creatine phosphate, phosphopyruvate, glyceraldehyde-3-phosphate and glucose-6-phosphate.

33. A method according to claim 32, wherein the at least two added energy sources are phosphoenolpyruvate and acetyl phosphate.

34. A method according to claim 33, wherein the at least two added energy sources comprise about 30mM phosphoenolpyruvate and about 60mM acetyl phosphate.

35. The method according to claim 28, wherein said enzyme is selected from the group consisting of a nuclease, a phosphatase and a polymerase.

36. A method for constructing an *in vitro* synthesis system as claimed in any of claims 14 to 20, said method comprising: obtaining at least one bacterial cell extract having a mutation resulting in reduced activity of at least one nuclease and adding to said bacterial cell extract Gam protein and at least two energy sources providing chemical energy for synthesis.

## Patentansprüche

1. Ein System zur *in vitro* Synthese zur Herstellung eines Proteins oder einer Nukleinsäure aus einem Nukleinsäure-Template, das einen Extrakt aus einer bakteriellen Zelle umfasst, wobei diese Zelle eine Mutation aufweist, die zu reduzierter Aktivität von zumindest einer Nuklease führt, **dadurch gekennzeichnet, dass** dieser Extrakt durch Hinzufügen von Gam-Protein modifiziert wird.

2. Ein System zur *in vitro* Synthese gemäß Anspruch 1, wobei es sich bei dem Gam-Protein um lösliches Gam-Protein handelt.

3. Das System zur *in vitro* Synthese gemäß Anspruch 1 oder 2, wobei es sich bei dem Template um ein DNA-Template und bei der Nuklease um eine DNase handelt.

4. Ein System zur *in vitro* Synthese gemäß Anspruch 3, wobei es sich bei der DNase um DNA-Exonuklease handelt.

5. Ein System zur *in vitro* Synthese gemäß Anspruch 3, wobei es sich bei der DNase um DNA-Endonuklease handelt.

6. Ein System zur *in vitro* Synthese gemäß Anspruch 5, wobei es sich bei der DNA-Endonuklease um Endonuklease A handelt.

7. Ein System zur *in vitro* Synthese gemäß Anspruch 1 oder 2, wobei es sich bei der Nuklease um eine RNase handelt.

8. Ein System zur *in vitro* Synthese gemäß Anspruch 7, wobei es sich bei der RNase um eine RNA-Exonuklease handelt.

9. Ein System zur *in vitro* Synthese gemäß Anspruch 7, wobei es sich bei der RNase um eine RNA-Endonuklease handelt.

10. Ein System zur *in vitro* Synthese gemäß Anspruch 9, wobei es sich bei der Endonuklease um RNase E handelt.

11. Ein System zur *in vitro* Synthese gemäß Anspruch 9, wobei es sich bei der Endonuklease um RNase I oder RNase I* handelt.

12. Ein System zur *in vitro* Synthese gemäß einem der vorhergehenden Ansprüche, das des Weiteren mindestens ein Nukleinsäure-Template ausgewählt aus der Gruppe bestehend aus einem DNA-Template und einem RNA-Template umfasst.

13. Ein System zur *in vitro* Synthese gemäß einem der vorhergehenden Ansprüche, das zumindest ein DNA-Template umfasst und wobei es sich bei dem System zur *in vitro* Synthese um ein *in vitro* Transkriptions-/Translationssystem handelt.

14. Ein System zur *in vitro* Synthese gemäß einem der vorhergehenden Ansprüche, das zumindest zwei hinzugefügte Energiequellen umfasst, die energiereiche Triphosphat-Verbindungen generiert oder regeneriert.

15. Ein System zur *in vitro* Synthese gemäß Anspruch 14, das zumindest zwei hinzugefügte Verbindungen, ausgewählt aus der Gruppe bestehend aus Pyruvat, Phosphoenolpyruvat (PEP), Carbamoylphosphat, Acetylphosphat, Creatinphosphat, Phosphopyruvat, Glyceraldehyd-3-Phosphat und Glucose-6-Phosphat umfasst.

16. Ein System zur *in vitro* Synthese gemäß Anspruch 15, das hinzugefügtes Acetylphosphat umfasst.

17. Ein System zur *in vitro* Synthese gemäß Anspruch 16, das des Weiteren hinzugefügtes Phosphoenolpyruvat umfasst.

18. Ein System zur *in vitro* Synthese gemäß Anspruch 17, das ungefähr 30 mM Phosphoenolpyruvat und ungefähr 60 mM Acetylphosphat umfasst.

19. Ein System zur *in vitro* Synthese gemäß einem der vorhergehenden Ansprüche, wobei der Extrakt aus der Zelle bezüglich der Aktivität von zumindest einem Enzym ausgewählt aus der Liste bestehend aus OmpT, RNase E, Alkaliner Phosphatase und Endonuklease I reduziert ist.

20. Ein System zur *in vitro* Synthese gemäß einem der vorhergehenden Ansprüche, wobei der Zellextrakt aus einer *Escherichia coli* Zelle stammt.

21. Eine Zusammensetzung, die folgendes umfasst:
a. einen Extrakt aus einer bakteriellen Zelle, wobei diese bakterielle Zelle zumindest eine Mutation aufweist, die zu reduzierter Aktivität einer Nuklease führt,
b. zumindest zwei hinzugefügte Energiequellen, welche die chemische Energie für die Synthese bereit stellen
c. hinzugefügtes Gam-Protein.

22. Eine Zusammensetzung gemäß Anspruch 21, die des Weiteren zumindest ein Nukleinsäure-Template in Anwesenheit von zumindest einem partiellen Syntheseprodukt des Templates umfasst.

23. Eine Zusammensetzung gemäß Anspruch 22, wobei es sich bei dem Produkt um ein Nukleinsäureprodukt handelt.

24. Eine Zusammensetzung gemäß Anspruch 23, wobei es sich bei dem Nukleinsäureprodukt um DNA handelt.

25. Eine Zusammensetzung gemäß Anspruch 23, wobei es sich bei dem Nukleinsäureprodukt um RNA handelt.

26. Ein Kit für *in vitro* Synthese, das folgendes umfasst:
a. einen Extrakt aus einer bakteriellen Zelle, wobei diese bakterielle Zelle zumindest eine Mutation aufweist, die zu reduzierter Aktivität einer Nuklease führt.
b. hinzugefügtes Gam-Protein
c. ein oder mehr als ein Reagens, ausgewählt aus der Liste bestehend aus Nukleotiden oder Derivaten von Nukleotiden, zumindest einer Polymerase, zumindest einem Co-Faktor, zumindest einem Puffer oder Puffersalz, zumindest einer Energiequelle für die Synthese, zumindest einem Nukleinsäure-Template, zumindest einem Reagens, um die Effizienz des Kits zu bestimmen.

27. Ein Kit gemäß Anspruch 26, das zumindest zwei oder mehr als zwei Energiequellen für die Synthese umfasst.

28. Ein Verfahren zur Herstellung von Protein oder Nukleinsäure aus einem Nukleinsäure-Template in einem *in vitro* System, das folgendes umfasst:
Kontaktieren des Templates mit
a. zumindest einem Extrakt aus einer bakteriellen Zelle, wobei diese Zelle eine Mutation aufweist, die zu reduzierter Aktivität von zumindest einer Nuklease führt,
b. Gam-Protein; und
c. zumindest zwei hinzugefügten Energiequellen, welche die chemische Energie für die Synthese bereit stellen;
wobei eine Mischung gebildet wird; und Inkubieren dieser Mischung unter Bedingungen, die ausreichen, um zumindest ein durch das Template kodiertes Protein herzustellen.

29. Ein Verfahren gemäß Anspruch 28, wobei die zumindest eine Nuklease ausgewählt wird aus der Gruppe bestehend aus RNAse E, RNAse I und Endonuklease I.

30. Ein Verfahren gemäß Anspruch 28 oder Anspruch 29, wobei es sich bei dem Gam-Protein-Inhibitor um lösliches Gam-Protein handelt.

31. Ein Verfahren gemäß einem der Ansprüche 28 bis 30, wobei jede der zumindest zwei hinzugefügten Energiequellen energiereiche Triphosphatverbindungen für die Proteinsynthese generiert oder regeneriert.

32. Ein Verfahren gemäß Anspruch 31, wobei die zumindest zwei hinzugefügten Energiequellen ausgewählt werden aus der Gruppe bestehend aus Pyruvat, Phosphoenolpyruvat (PEP), Carbamoylphosphat, Acetylphosphat, Creatinphosphat, Phosphopyruvat, Glyceraldehyd-3-Phosphat und Glucose-6-Phosphat.

33. Ein Verfahren gemäß Anspruch 32, wobei es sich bei den zumindest zwei hinzugefügten Energiequellen um Phosphoenolpyruvat und Acetylphosphat handelt.

34. Ein Verfahren gemäß Anspruch 33, wobei die zumindest zwei hinzugefügten Energiequellen ungefähr 30 mM Phosphoenolpyruvat und ungefähr 60 mM Acetylphosphat umfassen.

35. Das Verfahren gemäß Anspruch 28, wobei das Enzym ausgewählt wird aus der Gruppe bestehend aus einer Nuklease, einer Phosphatase und einer Polymerase.

36. Ein Verfahren zur Konstruktion eines Systems zur *in vitro* Synthese wie in einem der vorhergehenden Ansprüche 14 bis 20 beansprucht, wobei das Verfahren folgendes umfasst:
Erhalten von zumindest einem bakteriellen Extrakt, der eine Mutation aufweist, die zu reduzierter Aktivität von zumindest einer Nuklease führt; und
Hinzufügen von Gam-Protein und zumindest zwei Energiequellen, welche die chemische Energie für die Synthese bereit stellen, zu diesem bakteriellen Zellextrakt.

## Revendications

1. - Système de synthèse *in vitro* pour produire une protéine ou un acide nucléique à partir d'une matrice d'acide nucléique, comprenant un extrait provenant d'une cellule bactérienne, ladite cellule ayant une mutation conduisant à une activité réduite d'au moins une nucléase, **caractérisé par le fait que** ledit extrait est modifié par l'addition d'une protéine Gam.

2. - Système de synthèse *in vitro* selon la revendication 1, dans lequel la protéine Gam est une protéine Gam soluble.

3. - Système de synthèse *in vitro* selon la revendication 1 ou la revendication 2, dans lequel la matrice est une matrice d'ADN et le nucléase est une DNase.

4. - Système de synthèse *in vitro* selon la revendication 3, dans lequel la DNase est une ADN exonucléase.

5. - Système de synthèse *in vitro* selon la revendication 3, dans lequel la DNase est une ADN endonucléase.

6. - Système de synthèse *in vitro* selon la revendication 5, dans lequel l'ADN endonucléase est l'endonucléase A.

7. - Système de synthèse *in vitro* selon la revendication 1 ou la revendication 2, dans lequel la nucléase est une RNase.

8. - Système de synthèse *in vitro* selon la revendication 7, dans lequel la RNase est une ARN exonucléase.

9. - Système de synthèse *in vitro* selon la revendication 7, dans lequel la RNase est une ARN endonucléase.

10. - Système de synthèse *in vitro* selon la revendication 9, dans lequel l'endonucléase est la RNase E.

11. - Système de synthèse *in vitro* selon la revendication 9, dans lequel l'endonucléase est la RNase I ou la RNaseI*.

12. - Système de synthèse *in vitro* selon l'une quelconque des revendications précédentes, comprenant en outre au moins une matrice d'acide nucléique choisie dans le groupe constitué par une matrice d'ADN et une matrice d'ARN.

13. - Système de synthèse *in vitro* selon l'une quelconque des revendications précédentes, comprenant au moins une matrice d'ADN et dans lequel le système de synthèse *in vitro* est un système de transcription/traduction *in vitro.*

14. - Système de synthèse *in vitro* selon l'une quelconque des revendications précédentes, comprenant au moins deux sources d'énergie ajoutées qui génèrent ou régénèrent des composés triphosphates à haute énergie.

15. - Système de synthèse *in vitro* selon la revendication 14, comprenant au moins deux composés ajoutés choisis dans le groupe constitué par le pyruvate, le phosphoénolpyruvate (PEP), le phosphate de carbamoyle, le phosphate d'acétyle, le phosphate de créatine, le phosphopyruvate, le glycéraldéhyde-3-phosphate et le glucose-6-phosphate.

16. - Système de synthèse in *vitro* selon la revendication 15, comprenant du phosphate d'acétyle ajouté.

17. - Système de synthèse *in vitro* selon la revendication 16, comprenant en outre du phosphoénolpyruvate ajouté.

18. - Système de synthèse *in vitro* selon la revendication 17, comprenant du phosphoénolpyruvate environ 30mM et du phosphate d'acétyle environ 60mM.

19. - Système de synthèse *in vitro* selon l'une quelconque des revendications précédentes, dans lequel l'extrait provenant de ladite cellule est réduit dans l'activité d'au moins une enzyme choisie dans la liste constituée par OmpT, RNase E, phosphatase alcaline et endonucléase I.

20. - Système de synthèse *in vitro* selon l'une quelconque des revendications précédentes, dans lequel l'extrait cellulaire provient d'une cellule d'*Escherichia coli.*

21. - Composition comprenant :
a. un extrait provenant d'une cellule bactérienne, ladite cellule bactérienne ayant au moins une mutation conduisant à l'activité réduite d'une nucléase ;
b. au moins deux sources d'énergie ajoutées fournissant l'énergie chimique pour la synthèse ;
c. une protéine Gam ajoutée.

22. - Composition selon la revendication 21, comprenant en outre au moins une matrice d'acide nucléique en présence d'au moins un produit de synthèse partielle de ladite matrice.

23. - Composition selon la revendication 22, dans laquelle le produit est un produit d'acide nucléique.

24. - Composition selon la revendication 23, dans laquelle le produit d'acide nucléique est de l'ADN.

25. - Composition selon la revendication 23, dans laquelle le produit d'acide nucléique est de l'ARN.

26. - Kit pour une synthèse *in vitro*, comprenant :
a. un extrait provenant d'une cellule bactérienne, ladite cellule bactérienne ayant au moins une mutation conduisant à l'activité réduite d'une nucléase ;
b. une protéine Gam ajoutée ;
c. un ou plusieurs réactifs ajoutés choisis dans la liste constituée par des nucléotides ou dérivés de nucléotides, au moins une polymérase, au moins un co-facteur, au moins un tampon ou sel de tampon, au moins une source d'énergie pour la synthèse, au moins une matrice d'acide nucléique, au moins un réactif pour déterminer l'efficacité du kit.

27. - Kit selon la revendication 26, comprenant deux sources d'énergie ou davantage pour la synthèse.

28. - Procédé pour produire une protéine ou un acide nucléique à partir d'une matrice d'acide nucléique dans un système *in vitro* comprenant :
- la mise en contact de ladite matrice avec :
a. au moins un extrait provenant d'une cellule bactérienne, ladite cellule ayant une mutation conduisant à l'activité réduite d'au moins une nucléase ;
b. une protéine Gam ;
c. au moins deux sources d'énergie ajoutées fournissant l'énergie chimique pour la synthèse ;
- la formation d'un mélange ; et
- l'incubation dudit mélange dans des conditions suffisantes pour produire au moins une protéine codée par ladite matrice.

29. - Procédé selon la revendication 28, dans lequel la au moins une nucléase est choisie dans le groupe constitué par RNase E, RNase I et endonucléase I.

30. - Procédé selon la revendication 28 ou la revendication 29, dans lequel l'inhibiteur protéine Gam est une protéine Gam soluble.

31. - Procédé selon l'une quelconque des revendications 28 à 30, dans lequel chacune des au moins deux sources d'énergie ajoutées génère ou régénère des composés triphosphates à haute énergie en vue d'une synthèse protéique.

32. - Procédé selon la revendication 31, dans lequel les au moins deux sources d'énergie ajoutées sont choisies dans le groupe constitué par le pyruvate, le phosphoénolpyruvate (PEP), le phosphate de carbamoyle, le phosphate d'acétyle, le phosphate de créatine, le phosphopyruvate, le glycéraldéhyde-3-phosphate et le glucose-6-phosphate.

33. - Procédé selon la revendication 32, dans lequel les au moins deux sources d'énergie ajoutées sont le phosphoénolpyruvate et le phosphate d'acétyle.

34. - Procédé selon la revendication 33, dans lequel les au moins deux sources d'énergie ajoutées comprennent du phosphoénolpyruvate environ 30 mM et du phosphate d'acétyle environ 60 mM.

35. - Procédé selon la revendication 28, dans lequel ladite enzyme est choisie dans le groupe constitué par une nucléase, une phosphatase et une polymérase.

36. - Procédé pour construire un système de synthèse *in vitro* tel que revendiqué dans l'une quelconque des revendications 14 à 20, ledit procédé comprenant :
- l'obtention d'au moins un extrait de cellule bactérienne ayant une mutation conduisant à l'activité réduite d'au moins une nucléase ; et
- l'addition audit extrait de cellule bactérienne d'une protéine Gam et d'au moins deux sources d'énergie fournissant l'énergie chimique pour la synthèse.
